# EUROPÄISCHE PATENTSCHRIFT

(11) **EP 3 554 577 B1**
(45) Veröffentlichungstag und Bekanntmachung des Hinweises auf die Patenterteilung: **07.08.2024**
(21) Anmeldenummer: 17828682.9
(22) Anmeldetag: 14.12.2017
(51) Int. Cl.: A61M 1/16, A61M 1/34, A61M 1/36

(54) **SYSTEM ZUR EXTRAKORPORALEN BLUTBEHANDLUNG UND BEHANDLUNGSVORRICHTUNG**
SYSTEM FOR EXTRACORPOREAL BLOOD TREATMENT AND TREATMENT APPARATUS
SYSTÈME DE TRAITEMENT EXTRACORPOREL DU SANG ET DISPOSITIF DE TRAITEMENT

(30) Priorität: 15.12.2016 DE 102016014892; 16.06.2017 DE 102017210134
(43) Veröffentlichungstag der Anmeldung: 23.10.2019
(73) Patentinhaber: Fresenius Medical Care Deutschland GmbH, 61352 Bad Homburg (DE)
(72) Erfinder: RAMMO, Jörg, 66583 Spiesen-Elversberg (DE); KLEWINGHAUS, Jürgen, 61440 Oberursel (DE)
(74) Vertreter: Ricker, Mathias
(86) Internationale Anmeldenummer: PCT/EP2017/082781
(87) Internationale Veröffentlichungsnummer: WO 2018/109070

(56) Entgegenhaltungen:
- CN-A- 102 500 003
- CN-Y- 201 320 317
- DE-A1- 19 622 184
- US-A1- 2005 182 349
- US-A1- 2005 236 329
- US-A1- 2015 165 105

## Beschreibung

Die vorliegende Erfindung betrifft ein System zur extrakorporalen Blutbehandlung, wobei das System einen ersten Einlass zum Einbringen eines zu behandelnden Blutstroms in das System, wenigstens eine Blutbehandlungsvorrichtung sowie einen ersten Auslass zum Ausbringen eines behandelten Blutstroms aus dem System aufweist. Ferner betrifft die Erfindung eine Behandlungsvorrichtung mit einem solchen System. Weiter wird ein Kit, das die Komponenten des Systems umfasst, beschrieben. Des Weiteren wird ein Verfahren zum Betreiben eines solchen Systems und/oder einer Behandlungsvorrichtung mit einem solchen System beschrieben. Ferner wird ein Verfahren zur extrakorporalen Blutbehandlung mit einem solchen System oder mit einer solchen Behandlungsvorrichtung beschrieben.

Systeme zur extrakorporalen Blutbehandlung sind aus dem Stand der Technik grundsätzlich bekannt. Dabei sind auch Systeme bekannt, mit denen die Kombination zweier verschiedener Blutbehandlungen möglich ist. Entsprechende Verfahren zur extrakorporalen Blutbehandlung mit vorgenannten Systemen sind ebenfalls bekannt.

Aus der WO 2015/067232 A1 ist beispielsweise ein System zur Peritoneal-Dialyse mit einem in einem extrakorporalen Kreislauf angeordneten Oxygenator bekannt.

In der DE 196 22 184 A1 ist ein Multifunktionsgerät zur multifunktionalen, extrakorporalen Blutbehandlung beschrieben, mit welchem sowohl eine Gasaustauschbehandlung als auch eine Dialysebehandlung möglich ist.

In der US 2005/182349 A1 wird ein Behandlungssystem offenbart aufweisend Blutführungsleitungen und eine UF-Einheit zur Erzeugung von Ultrafiltrat stromabwärts oder stromaufwärts zu einer CVVH-Hämofiltrationseinheit. Eine Ultrafiltrations-Bypass-Leitung umfasst einen Oxygenator, eine Zentrifugalpumpe und eine bioartifizielle Leber.

Ferner ist bekannt, eine extrakorporale Dialysebehandlung zur kontinuierlichen Nierenersatz-Therapie (CRRT = Continuous Renal Replacement Therapy) in einem gemeinsamen extrakorporalen Blutkreislauf mit einer extrakorporalen Adsorptionsbehandlung zur Sepsis-Therapie zu kombinieren, beispielsweise aus der EP 2 735 326 A1 oder der EP 0 236 0 509 B1.

Die EP 2 735 326 A1 offenbart ein System zur Blutbehandlung, welches zwei, jeweils Hohlfasermembranen enthaltende Dialysatoren aufweist, die dazu vorgesehen sind, in einem extrakorporalen Blutkreislauf zur Blutbehandlung in Reihe geschaltet zu werden und nacheinander durchströmt zu werden, wobei einer der beiden Dialysatoren ein adsorbierendes Material aufweist.

Die EP 0 236 0 509 B1 lehrt ebenfalls, zur Kombination von Dialysebehandlung und Adsorptionsbehandlung einen Dialysator und einen Adsorber in einem extrakorporalen Blutkreislauf in Reihe geschaltet anzuordnen.

Ferner ist bekannt, eine extrakorporale Dialysebehandlung zur kontinuierlichen Nierenersatz-Therapie in einem gemeinsamen extrakorporalen Blutkreislauf mit einer extrakorporalen Gasaustauschbehandlung zur CO2-Entfernung aus dem Blut (ECCO2R ₌ ExtraCorporeal CO2-Removal) oder zur extrakorporalen Beatmung mit gleichzeitiger Sauerstoff-Anreicherung (ECMO = ExtraCorporeal MembraneOxygenation) zu kombinieren.

Darüber hinaus ist aus der EP 2 461 847 B1 eine Blutbehandlungsvorrichtung mit einer Gasaustauschvorrichtung bekannt, die neben der Gasaustauschbehandlung eine Adsorptionsbehandlung ermöglicht, wobei die Gasaustauschvorrichtung dazu einen Träger umfasst, der mit Substanzen zur adsorptiven Entfernung von im Blut, Blutersatzmitteln oder Lösungen zum Einbringen in den menschlichen und/oder tierischen Blutkreislauf befindlichen Toxinen biologischer und chemisch-synthetischer Herkunft, deren Metaboliten und Abbauprodukten beschichtet ist.

Vor diesem Hintergrund ist es eine Aufgabe der Erfindung, ein verbessertes System zur extrakorporalen Blutbehandlung bereitzustellen, insbesondere ein System zur extrakorporalen Blutbehandlung, mit welchem die Möglichkeiten der extrakorporalen Blutbehandlung erweitert werden können und welches zusätzliche Behandlungsmöglichkeiten bietet, insbesondere jeweils flexibel in Abhängigkeit vom jeweiligen Behandlungsfall, ohne einen weiteren Patientenzugang legen zu müssen oder einen weiteren, zusätzlichen extrakorporalen Blutkreislauf herstellen zu müssen. Ferner ist es eine Aufgabe der Erfindung eine entsprechende Behandlungsvorrichtung, bereitzustellen.

Diese Aufgaben werden erfindungsgemäß durch ein System zur extrakorporalen Blutbehandlung mit den Merkmalen von Anspruch 1 und durch eine Behandlungsvorrichtung mit den Merkmalen von Anspruch 14 gelöst. Vorteilhafte Ausführungen der Erfindung sind Gegenstand der abhängigen Ansprüche, der Beschreibung und der Figuren und werden im Folgenden näher erläutert.

Ein erfindungsgemäßes System zur extrakorporalen Blutbehandlung weist einen ersten Einlass zum Einbringen eines zu behandelnden Blutstroms in das System, wenigstens eine erste Blutbehandlungsvorrichtung, eine zweite Blutbehandlungsvorrichtung, eine dritte Blutbehandlungsvorrichtung und einen ersten Auslass zum Ausbringen eines behandelten Blutstroms aus dem System auf.

Die erste Blutbehandlungsvorrichtung weist eine Adsorbervorrichtung zur Entfernung wenigstens eines körperfremden und/oder wenigstens eines körpereigenen Pathogens und/oder eine Plasmatrennvorrichtung zum Trennen von Blutplasma von den übrigen Blutbestandteilen auf oder ist eine Adsorbervorrichtung und/oder eine Plasmatrennvorrichtung.

Die zweite Blutbehandlungsvorrichtung ist als Dialysevorrichtung ausgebildet, insbesondere als Dialysevorrichtung zur Nierenersatztherapie, vorzugsweise zur kontinuierlichen Nierenersatztherapie.

Die dritte Blutbehandlungsvorrichtung ist als Gasaustauschvorrichtung ausgebildet, insbesondere als Gasaustauschvorrichtung zur zumindest teilweisen CO2-Entfernung aus einem die Gasaustauschvorrichtung durchströmenden Blutstrom und/oder zur Zufuhr eines Gases oder Gasgemisches in einen die Gasaustauschvorrichtung durchströmenden Blutstrom.

Dabei sind die erste, die zweite und die dritte Blutbehandlungsvorrichtung in einem funktionsgemäßen Anwendungszustand des Systems bezogen auf eine Blutflussrichtung eines zu behandelnden Blutstroms zwischen dem ersten Einlass und dem ersten Auslass des Systems sequenziell in Reihe geschaltet und nacheinander von einem zu behandelnden Blutstrom extrakorporal durchströmbar. Die Reihenfolge der Anordnung der Blutbehandlungsvorrichtungen richtet sich dabei vorzugsweise nach dem jeweiligen Anwendungsfall.

Unter einer extrakorporalen Blutbehandlung im Sinne der vorliegenden Erfindung wird eine Blutbehandlung außerhalb eines menschlichen oder tierischen Körpers verstanden, wobei die extrakorporale Blutbehandlung als solche aus dem Stand der Technik grundsätzlich bekannt ist.

Unter einem Blutstrom im Sinne der vorliegenden Erfindung wird ein Massenstrom von Vollblut verstanden.

Als Blutbehandlungsvorrichtung im Sinne der Erfindung wird eine Vorrichtung verstanden, mit welcher eine Blutmasse, insbesondere ein Blutstrom, behandelt werden kann, d.h. in seiner Zusammensetzung verändert werden kann.

Zur extrakorporalen Blutbehandlung kann einem erfindungsgemäßen System über den ersten Einlass ein zu behandelnder Blutstrom, nämlich zu behandelndes Blut, d.h. sogenanntes Vollblut, das sämtliche üblicherweise im Blut vorhandenen Bestandteile enthält, zugeführt werden sowie über den ersten Auslass des Systems aus dem System abgeführt werden.

In einer Ausgestaltung eines erfindungsgemäßen Systems kann der zu behandelnde Blutstrom aus einem Speichervolumen zugeführt werden, wie beispielsweise aus einem Konservierungsbeutel, d.h. aus einer Blutkonserve oder dergleichen, und/oder dem System direkt von einem zu behandelnden Patienten oder zu behandelndem Tier zugeführt werden.

Der behandelte Blutstrom kann in ein Speichervolumen abgeführt werden, beispielsweise ebenfalls in einen entsprechenden Konservierungsbeutel oder dergleichen, und/oder einem separierten Transplantationsorgan zur Versorgung zugeführt werden und/oder direkt einem zu behandelnden Patienten oder einem zu behandelnden Tier zugeführt werden.

Besonders bevorzugt ist ein erfindungsgemäßes System zur extrakorporalen Blutbehandlung dazu ausgebildet, in den menschlichen und/oder tierischen Blutkreislauf eingebracht zu werden und insbesondere mit dem intrakorporalen Blutkreislauf eines zu behandelnden Patienten oder Tieres unter Herstellung eines extrakorporalen Blutkreislaufes verbunden zu werden.

In einer bevorzugten Ausgestaltung eines erfindungsgemäßen Systems ist das System dazu ausgebildet, veno-venös (VV) mit dem intrakorporalen Blutkreislauf eines Patienten oder eines Tieres verbunden zu werden oder arterio-venös (AV). Je nach Anwendungsfall kann die veno-venöse oder die arterio-venöse Verbindung eines erfindungsgemäßen Systems mit dem intrakorporalen Blutkreislaufs eines zu behandelnden Patienten oder zu behandelnden Tieres vorteilhafter sein. Dies richtet sich insbesondere nach der erforderlichen Blutbehandlung bzw. den erforderlichen Blutbehandlungen bzw. der erforderlichen Kombination von Blutbehandlungen. In einer weiteren Ausgestaltung kann das erfindungsgemäße System auch über einen oder mehrere künstlich angelegte Blutzugänge, wie beispielsweise eine Fistel oder ein Shunt, mit dem intrakorporalen Blutkreislauf eines Patienten oder Tieres verbunden werden.

Bei einem erfindungsgemäßen System zur extrakorporalen Blutbehandlung sind grundsätzlich alle Varianten bezüglich der Reihenfolge der Anordnung der einzelnen Blutbehandlungsvorrichtungen in Blutflussrichtung möglich, wobei insgesamt 3!=6 Möglichkeiten der Anordnung bestehen, wobei einige dieser Anordnungsmöglichkeiten besondere Vorteile aufweisen, welche im weiteren Verlauf dieser Anmeldung näher erläutert werden.

Vorzugsweise sind der erste Einlass und/oder der erste Auslass eines erfindungsgemäßen Systems durch Schlauchleitungen mit vorzugsweise jeweils wenigstens einem entsprechenden Anschluss gebildet bzw. weisen vorzugsweise jeweils eine oder mehrere entsprechende Schlauchleitungen auf, welche insbesondere jeweils über einen geeigneten Zugang mit einem Blutgefäß eines zu behandelnden Patienten oder Tieres und/oder einem Speichervolumen verbunden werden können. Die Schlauchleitungen eines erfindungsgemäßen Systems können dabei ein Schlauchset bilden, insbesondere ein austauschbares Schlauchset.

Bevorzugt weist ein erfindungsgemäßes System zur extrakorporalen Blutbehandlung eine Zuführleitung zum Einbringen, insbesondere zur Zufuhr, eines von einem Patienten und/oder eines von einem Tier und/oder einem Speichervolumen entnommenen Blutstroms in das System und/oder eine Rückführleitung zum Ausbringen, insbesondere zur Abfuhr eines behandelten Blutstroms aus dem System und/oder zur Rückführung des behandelten Blutstroms oder eines Teils davon in den intrakorporalen Blutkreislaufs eines Patienten oder zu behandelnden Tieres und/oder in ein Speichervolumen oder in ein separiertes Transplantationsorgan auf.

In einer bevorzugten Ausgestaltung eines erfindungsgemäßen Systems weist das System in der Rückführleitung wenigstens ein Absperrventil auf, um eine Abfuhr bzw. das Ausbringen des behandelten Blutstromes aus dem System sperren zu können, insbesondere um eine Rückführung in den intrakorporalen Blutkreislauf eines zu behandelnden Patienten oder Tieres verhindern zu können. Darüber hinaus kann die Rückführleitung eine Schutzvorrichtung aufweisen, wie beispielsweise einen Filter oder eine magnetische Vorrichtung, um unerwünschte Partikel zurückzuhalten und insbesondere einen Übertritt dieser unerwünschten Partikel in den intrakorporalen Blutkreislauf zu vermeiden und/oder zu verhindern.

Im Fall einer Sepsis ist oftmals, insbesondere bei Intensivpatienten, neben einer kontinuierlichen Nierenersatz-Therapie aufgrund von Nierenversagen häufig gleichzeitig eine Beatmung angezeigt. Mit einem erfindungsgemäßen System können gleichzeitig und mit nur einem extrakorporalen Blutkreislauf wenigstens drei Blutbehandlungen durchgeführt werden, nämlich eine Dialysebehandlung, eine Adsorptionsbehandlung und/oder eine Plasmatrennung und eine extrakorporale Gasaustauschbehandlung, insbesondere eine extrakorporale Beatmung anstelle oder in Kombination mit einer mechanischen Beatmung, die eine Intubation oder eine Tracheotomie erfordert.

Die extrakorporale Gasaustauschbehandlung hat gegenüber der mechanischen Beatmung den Vorteil, dass die Lunge entlastet werden kann und sich dadurch besser regenerieren kann, da der erforderliche Gasaustausch über die Gasaustauschvorrichtung erfolgt und nicht wie bei der mechanischen Beatmung weiter über die Lunge. Daher ist insbesondere in Fällen, in welchen die Lunge angegriffen ist, wie es gerade bei einer Sepsis häufig vorkommt, eine extrakorporale Beatmung mittels einer Gasaustauschvorrichtung vorteilhafter als die mechanische Beatmung. Bei Kombination mit einer mechanischen Beatmung kann letztere weniger intensiv ausgeführt werden, was den Patienten weniger belastet.

Durch die erfindungsgemäße Reihenschaltung von Adsorbervorrichtung und/oder Plasmatrennvorrichtung, Dialysevorrichtung und Gasaustauschvorrichtung ist ferner nur ein extrakorporaler Blutkreislauf erforderlich. Infolgedessen können mit einem erfindungsgemäßen System auch Patienten gleichzeitig einer extrakorporalen Blutbehandlung umfassend eine Adsorptionsbehandlung und/oder eine Plasmatrennung, insbesondere eine Plasmabehandlung, eine Dialysebehandlung und eine Gasaustauschbehandlung unterzogen werden, deren Stabilität nicht zur gleichzeitigen Versorgung zweier extrakorporaler Blutkreisläufe ausreichend ist oder deren Zustand eine zeitversetzte extrakorporale Blutbehandlung nicht zulässt. Ferner sind bei nur einem extrakorporalen Blutkreislauf weniger Zugänge erforderlich. Dadurch reduzieren sich die Belastungen für einen Patienten sowie die Infektionsgefahr.

Ein erfindungsgemäßes System ermöglicht durch die sequenzielle Anordnung einer Adsorbervorrichtung und/oder einer Plasmatrennvorrichtung, einer Dialysevorrichtung sowie einer Gasaustauschvorrichtung gleichzeitig beispielsweise die Behandlung einer Sepsis, eine Dialysebehandlung bei eingeschränkter Nierenfunktion oder Nierenversagen sowie eine extrakorporale Beatmung.

Eine Adsorbervorrichtung im Sinne der vorliegenden Erfindung ist eine Vorrichtung, die dazu ausgebildet ist, einen oder mehrere Bestandteile des die Adsorbervorrichtung durchströmenden Blutstroms mittels Adsorption aus dem Blutstrom zu entfernen. Adsorbervorrichtungen sind aus dem Stand der Technik grundsätzlich bekannt.

In einer vorteilhaften Ausgestaltung eines erfindungsgemäßen Systems ist die erste Blutbehandlungsvorrichtung eine Adsorbervorrichtung, insbesondere eine zur Endotoxinadsorption, zur Zytokinadsorption und/oder zur Immunadsorption ausgebildete Adsorbervorrichtung oder weist eine derartige Adsorbervorrichtung auf. Insbesondere ist die Adsorbervorrichtung zur Entfernung wenigstens eines körperfremden Pathogens ausgebildet, beispielsweise zur Entfernung wenigstens eines Medikaments und/oder wenigstens eines Arzneimittelwirkstoffs und/oder wenigstens eines Pflanzengiftes und/oder eines organischen Giftes und/oder einer anderen toxischen Substanz und/oder von Bakterien, Viren, Pilzen und/oder anderen Organismen und/oder zur Entfernung wenigstens eines körpereigenen Pathogens, beispielsweise zur Entfernung von und/oder wenigstens eines Immunkomplexes und/oder wenigstens eines Immunglobulins und/oder wenigstens einer Substanzen der infllammatorischen Antwort des Körpers (Mediator) und/oder von Antikörpern und/oder zur Entfernung wenigstens eines sogenannten pathogen-assoziierten molekularen Musters ("pathogen associated molecular patterns" - PAMPs) und/oder wenigstens eines sogenannten Alarmins ("danger or damage associated molecular patterns" - DAMPs).

Eine Plasmatrennvorrichtung im Sinne der vorliegenden Erfindung ist eine Vorrichtung, mittels welcher Blutplasma einer in die Plasmatrennvorrichtung eingebrachten Blutmenge zumindest teilweise von den übrigen Bestandteilen der Blutmenge getrennt werden kann. Eine Plasmatrennvorrichtung weist insbesondere einen Plasmafilter und/oder eine Zentrifugiereinrichtung auf oder ist als Plasmafilter oder Zentrifuge ausgebildet.

Die Dialysevorrichtung eines erfindungsgemäßen Systems zur extrakorporalen Blutbehandlung ist bevorzugt zur Durchführung wenigstens eines Verfahrens aus einer Gruppe verschiedener Verfahren zur Blutreinigung ausgebildet, vorzugsweise zur kontinuierlichen Nierenersatztherapie, d.h. zur CRRT-Behandlung insbesondere zur Hämodialyse, Hämofiltration, Hämodialfiltration, Hämoperfusion und/oder zur Peritoneladialyse, wobei derartige Dialysevorrichtungen sowie die zugehörigen Dialyseverfahren aus dem Stand der Technik ebenfalls grundsätzlich bekannt sind.

Die Gasaustauschvorrichtung eines erfindungsgemäßen Systems ist vorzugsweise zur zumindest teilweisen CO2-Entfernung und/oder zur Anreicherung eines Blutstroms mit einem Gas und/oder mit einem Gasgemisch ausgebildet, insbesondere zur Anreicherung mit Sauerstoff (02) und/oder mit einem medizinischen Gasgemisch, wobei insbesondere Gasaustauscher, die zur CO2-Entfernung und zur Anreicherung eines Blutstroms mit 02 oder einem Gasgemisch ausgebildet sind (sogenannte "Oxygenatoren"), grundsätzlich aus dem Stand der Technik bekannt sind. Häufig werden sogenannte Membranoxygenatoren eingesetzt, bei deren Verwendung die extrakorporale Gasaustauschbehandlung üblicherweise als extrakorporale Membranoxygenierung (ECMO = Extra Corporeal Membrane Oxygenation) bezeichnet wird.

In einer bevorzugten Ausgestaltung eines erfindungsgemäßen Systems weist das System, insbesondere die Gasaustauschvorrichtung, einen Oxygenator der Firma Novalung GmbH oder der Medos Medizintechnik AG auf, insbesondere einen unter der Bezeichnung iLA^{®} vertriebenen Oxygenator der Novalung GmbH oder einen aus der sogenannten "hilite"-Produktfamilie der Medos Medizintechnik AG.

In einer bevorzugten Ausgestaltung eines erfindungsgemäßen Systems weist das System, insbesondere die Gasaustauschvorrichtung, einen gemäß eines in der DE 10 2005 039 446 A1 ausgebildeten Oxygenator auf oder besteht aus einem solchen Oxygenator mit einer integrierten Blutpumpe zur Förderung des zu behandelnden Blutstroms durch den Oxygenator hindurch.

In einer vorteilhaften Ausgestaltung eines erfindungsgemäßen Systems zur extrakorporalen Blutbehandlung weist die Gasaustauschvorrichtung einen Membranoxygenator auf, der zumindest teilweise, vorzugsweise vollständig, wie in der DE 10 2008 045 621 A1 beschrieben oder in der EP 3 090 769 A1 oder der EP 3 090 768 A1 beschrieben ausgebildet ist, oder besteht aus einem solchen Oxygenator.

Besonders bevorzugt weist ein erfindungsgemäßes System, insbesondere die Gasaustauschvorrichtung eines erfindungsgemäßen Systems, einen plattenförmigen Membranoxygenator mit einem Membranmodul mit schichtweise übereinander angeordneten Hohlfasermembranmatten auf, wobei vorzugsweise wenigstens zwei übereinanderliegende Matten um einem definierten Winkel zueinander versetzt angeordnet sind, insbesondere um einen Winkel von 90° versetzt zueinander, bezogen auf eine mittlere Längserstreckung der einzelnen Hohlfasern der Hohlfasermembranmatten. Besonders bevorzugt sind die einzelnen Hohlfasermembranmatten dabei derart miteinander verpottet, dass der Oxygenator einen rechteckigen, insbesondere quadratischen, oder einen runden, insbesondere kreisrunden, Durchströmquerschnitt aufweist.

Ein derartiger, plattenförmiger Membranoxygenator hat den Vorteil, dass sich eine besonders vorteilhafte Durchströmung einstellt und der entstehende Druckabfall besonders klein gehalten werden kann, in einigen Fällen kleiner als 5 mmHg. Dies kann in einigen Behandlungsfällen besonders vorteilhaft sein, insbesondere in Behandlungsfällen, in denen die Kombination einer extrakorporalen Adsorptionsbehandlung mit einer extrakorporalen Dialysebehandlung und einer extrakorporalen Dialysebehandlung angezeigt ist.

Da in den meisten Anwendungsfällen die zur Förderung des zu behandelnden Blutstroms durch das System hindurch zur Verfügung stehende Pumpleistung des Herzens oder einer implantierten Pumpe eines zu behandelnden Patienten oder eines zu behandelnden Tieres nicht ausreicht oder ein veno-venöser Zugang zur Herstellung des extrakorporalen Blutkreislaufs zur extrakorporalen Blutbehandlung mit einem erfindungsgemäßen System vorteilhafter ist, d.h. die Entnahme des zu behandelnden Blutstroms erfolgt aus einer Vene und die Rückführung des behandelten Blutstroms erfolgt ebenfalls in eine Vene, weist ein erfindungsgemäßes System in einer vorteilhaften Ausgestaltung wenigstens eine erste Pumpe, insbesondere eine als Blutpumpe ausgebildete erste Pumpe, zur Förderung wenigstens eines Anteils eines zu behandelnden Blutstroms auf, wobei die erste Pumpe vorzugweise in Blutflussrichtung zwischen dem ersten Einlass und der ersten Blutbehandlungsvorrichtung in Blutflussrichtung angeordnet ist und insbesondere zur Förderung des gesamten zu behandelnden Blutstroms ausgebildet ist. D.h. in einer vorteilhaften Ausgestaltung eines erfindungsgemäßen Systems ist die erste Pumpe vorzugsweise in Reihe mit den drei Blutbehandlungsvorrichtungen geschaltet und insbesondere bevorzugt unmittelbar nach dem ersten Einlass angeordnet und vor der ersten Blutbehandlungsvorrichtung bezogen auf die Blutflussrichtung.

In einigen Fällen kann es vorteilhafter sein, wenn die erste Pumpe zwischen der ersten Blutbehandlungsvorrichtung und der zweiten Blutbehandlungsvorrichtung angeordnet ist oder zwischen der zweiten Blutbehandlungsvorrichtung und der dritten Blutbehandlungsvorrichtung oder sogar erst nach der dritten Blutbehandlungsvorrichtung, jeweils bezogen auf die Blutflussrichtung bei einer funktionsgemäßen Verwendung des erfindungsgemäßen Systems.

In einer besonders bevorzugten Ausgestaltung weist ein erfindungsgemäßes System mehrere Pumpen, insbesondere mehrere jeweils als Blutpumpe ausgebildete Pumpen zur Förderung des zu behandelnden Blutstroms auf, welche vorzugsweise in Blutflussrichtung in Reihe mit den Behandlungsvorrichtungen geschaltet sind und insbesondere derart in Blutflussrichtung angeordnet sind, dass sich jeweils die für eine optimale Behandlung erforderlichen Druckverhältnisse an einer oder mehreren der Blutbehandlungsvorrichtungen einstellen.

In einer bevorzugten Ausgestaltung weist ein erfindungsgemäßes System als erste Pumpe eine Schlauchrollenpumpe auf. In einer besonders bevorzugten Ausgestaltung weist das erfindungsgemäße System als erste Pumpe eine Zentrifugalpumpe auf. Diese kann eine als Rotorpumpe ausgebildete Diagonalpumpe sein, wobei die Pumpe vorzugsweise einen von einem Antriebsteil entkoppelten blutführenden Teil aufweist und der Rotor der Pumpe insbesondere über eine vorzugsweise auf einem Dorn gelagerte Lagerkugel insbesondere aus Keramik oder Aluminiumoxid gelagert ist und an seiner, dem Antriebsteil zugewandten Unterseite vorzugsweise Permanentmagneten aufweist und mittels magnetischer Kopplung antreibbar ist. Besonders bevorzugt ist wenigstens eine Pumpe dabei derart ausgebildet, dass beim Durchströmen der Pumpe nur geringe Scherspannungen entstehen und so die einzelnen Blutbestandteile, insbesondere die roten Blutkörperchen möglichst wenig geschädigt werden.

In einer besonders bevorzugten Ausgestaltung weist ein erfindungsgemäßes System eine gemäß der in der DE 10 2010 024 650 A1 beschriebenen Blutpumpe ausgebildete Pumpe auf. Vorzugsweise ist die Größe der Blutpumpe, insbesondere deren Anschlussgeometrie, dabei derart gewählt, dass sie an den jeweiligen zu fördernden Blutstrom bzw. an die Größe und/oder das Blutvolumens des zu behandelnden Patienten oder Tieres angepasst ist,

Da die Effektivität einer Hämofiltrations-Blutbehandlung von einem am Hämofilter anliegenden Druckgradienten abhängt, insbesondere von einem hydrostatischen Druckgefälle zwischen den beiden Seiten der Filtermembran, dem sogenannten Transmembrandruck (TMP), ist es für ein optimales Hämofiltration-Blutbehandlungs-Ergebnis vorteilhaft, wenn ein zur Hämofiltration oder Hämodiafiltration ausgebildetes erfindungsgemäßes System zur extrakorporalen Blutbehandlung und/oder eine zur Hämofiltration oder Hämodiafiltration ausgebildete Dialysevorrichtung eines erfindungsgemäßen Systems wenigstens eine Blutpumpe zur Förderung wenigstens eines Teils des zu behandelnden Blutstroms aufweist, mittels derer durch eine Veränderung des Blutstromflusses ein sich an wenigstens einer der Blutbehandlungsvorrichtungen einstellender Druck und/oder ein sich einstellender resultierender Druckgradient, insbesondere ein sich ergebender Transmembrandruck an der Dialysemembran der Dialysevorrichtung, definiert einstellbar ist.

Bevorzugt weist ein erfindungsgemäßes System zur Einstellung eines definierten Transmembrandrucks mehrere entsprechend ausgebildete und ansteuerbare Pumpen auf, insbesondere verschiedene, entsprechend ausgebildete und entsprechend ansteuerbare Pumpen, wie beispielsweise eine oder mehrere Blut-, Dialysat-, Filtrat-, und/oder Substituatpumpen, mit welchen der Blutstromfluss derart eingestellt werden kann, dass sich ein gewünschter, definierter Transmembrandruck am Hämofilter der Dialysevorrichtung eergibt.

In einer weiteren vorteilhaften Ausgestaltung eines erfindungsgemäßen Systems weist das System einen weiteren, insbesondere zweiten, Einlass zur Zugabe einer ersten Zusammensetzung in den Blutstrom auf, insbesondere in den zu behandelnden Blutstrom, wobei dieser weitere Einlass vorzugweise in Blutflussrichtung derart angeordnet ist, dass die erste Zusammensetzung dem Blutstrom in Blutflussrichtung vor der ersten Pumpe zuführbar ist und/oder vor der ersten der drei Blutbehandlungsvorrichtungen, insbesondere vor der Adsorbervorrichtung.

Bevorzugt ist dieser weitere, insbesondere zweite, Einlass dabei zur Zugabe eines flüssigen Antikoagulants ausgebildet, insbesondere zur Zugabe einer antikoagulierend wirkenden Citrat-Lösung. Dabei ist ein erfindungsgemäßes System besonders bevorzugt derart ausgebildet, dass die erste Zusammensetzung, insbesondere ein Antikoagulant, spätestens vor einer Behandlungsstrecke der ersten Behandlungseinrichtung, d.h. stromaufwärts von der ersten Behandlungseinrichtung, dem Blutstrom zugeführt werden kann. Besonders vorteilhaft ist der zweite Einlass dabei derart angeordnet, dass die Zugabe stromaufwärts der ersten Pumpe, d.h. in Blutflussrichtung vor der ersten Pumpe, erfolgen kann. Dadurch kann das Clotting-Risiko in innerhalb des Systems, insbesondere innerhalb einer weiter stromabwärts angeordneten Adsorbervorrichtung, reduziert werden.

Als Clotting bezeichnet man die Koagulation von Blutbestandteilen, d.h. ein Verklumpen von Blutbestandteilen.

Als Behandlungsstrecke im Sinne der Erfindung wird ein Strömungsweg bezeichnet, entlang dem eine tatsächliche Blutbehandlung erfolgt.

In einigen Fällen hat sich als vorteilhaft erwiesen, wenn ein erfindungsgemäßes System eine weitere, insbesondere zweite, separate Pumpe zur Förderung der ersten Zusammensetzung aufweist, insbesondere zur Förderung der ersten Zusammensetzung aus einem ersten Speichervolumen, in welchem die erste Zusammensetzung aufgenommen ist, heraus in den zu behandelnden Blutstrom. Das Speichervolumen, in dem die erste Zusammensetzung vorzugsweise aufgenommen ist, ist insbesondere ein Konservierungsbeutel oder ein entsprechend vergleichbar ausgebildetes Behältnis zur Aufbewahrung der ersten Zusammensetzung, insbesondere ein Behältnis, das eine sterile Aufbewahrung bei ausreichender Haltbarkeit der Zusammensetzung ermöglicht.

Da bei der Hämofiltration und der Hämodiafiltration üblicherweise nicht nur Moleküle, von denen der zu behandelnde Blutstrom zu reinigen ist, durch die Filtermembran, den sogenannten Hämofilter, der Dialysevorrichtung hindurchtreten können, sondern auch ein Teil der Plasmaflüssigkeit durch den Hämofilter hindurchtritt und als Effluent mitabgeführt wird, ist es in diesen Fällen in der Regel erforderlich, d.h. insbesondere in denjenigen Fällen, in denen die Dialysevorrichtung zur Hämofiltration oder zur Hämodiafiltration ausgebildet ist, dem Blutstrom zum Ausgleich des dadurch entstehenden Flüssigkeitsverlustes ein Substituat, in der Regel eine physiologische Substitutionsflüssigkeit, insbesondere eine Elektrolytlösung, zuzuführen. Das Substituat kann dabei grundsätzlich vor und/oder nach der Dialysebehandlung zugeführt werden. In manchen dieser Anwendungsfälle ist es vorteilhafter, das Substituat erst nach der Dialysebehandlung dem Blutstrom zuzuführen, d.h. insbesondere erst nach dem Hämofilter, wobei in einigen Fällen eine Zufuhr erst unmittelbar vor der Rückführung des behandelten Blutmassenstroms in den intrakorporalen Blutkreislauf eines zu behandelnden Patienten oder Tieres besonders vorteilhaft ist.

Bei der Hämodialyse, d.h. wenn die Dialysevorrichtung zur Hämodialyse oder zur Hämodiafiltration ausgebildet ist, kann, insbesondere bei einer Zugabe einer antikoagulierend wirkenden Citrat-Lösung in den Blutstrom, dem Blutstrom über das Effluent Kalzium entzogen werden, was ebenfalls auszugleichen ist, wobei ein auf diese Weise entstandener Kalzium-Verlust bevorzugt stromabwärts der Dialysevorrichtung ausgeglichen wird, insbesondere erst nach der letzten Blutbehandlungsvorrichtung.

In einer weiteren vorteilhaften Ausgestaltung eines erfindungsgemäßen Systems weist ein erfindungsgemäßes System daher einen weiteren, insbesondere dritten, Einlass zur Zugabe einer zweiten Zusammensetzung in den Blutmassenstrom auf, insbesondere zur Zugabe einer zweiten Zusammensetzung in den behandelten Blutmassenstrom, wobei dieser weitere Einlass vorzugsweise in Blutflussrichtung derart angeordnet ist, dass die zweite Zusammensetzung dem Blutmassenstrom in Blutflussrichtung nach der Dialysevorrichtung zuführbar ist, insbesondere nach der letzten Blutbehandlungsvorrichtung.

Dabei ist dieser weitere, insbesondere dritte, Einlass insbesondere zur Zugabe einer zweiten Zusammensetzung in Form eines Substituats zum Ausgleich eines bei der Hämofiltration oder Hämodiafiltration entstehenden Flüssigkeitsverlusts und/oder zur Zugabe einer zweiten Zusammensetzung in Form einer flüssigen Kalzium-Lösung zum Ausgleich eines bei einer Hämodialyse entstehenden Kalzium-Verlusts ausgebildet.

D.h. mit anderen Worten, dass bei einer bevorzugten Ausgestaltung eines erfindungsgemäßen Systems zur extrakorporalen Blutbehandlung, vorzugsweise nach der Behandlungsstrecke der Dialysevorrichtung und oder nach einem Dialysevorgang dem Blutmassenstrom eine zweite Zusammensetzung zugeführt werden kann. Besonders bevorzugt kann die zweite Zusammensetzung dem Blutstrom dabei unmittelbar vor der Rückführung in den intrakorporalen Blutkreislauf zugeführt werden, insbesondere unmittelbar in die Rückführungsleitung eingeleitet werden.

In einigen Fällen hat sich als vorteilhaft herausgestellt, wenn ein erfindungsgemäßes System zur Förderung der zweiten Zusammensetzung eine weitere Pumpe aufweist, insbesondere eine dritte Pumpe, welche insbesondere zur Förderung der zweiten Zusammensetzung aus einem zweiten Speichervolumen, in welchem die zweite Zusammensetzung aufgenommen ist, in den Blutstrom ausgebildet ist.

In einigen Fällen kann es aber auch vorteilhaft sein, einen weiteren, insbesondere separaten, Einlass zur Zufuhr des erforderlichen Substituats zum Ausgleich eines in der Dialysevorrichtung bei der Hämofiltration oder Hämodiafiltration entstehenden Flüssigkeits- bzw. Volumenverlustes vorzusehen, insbesondere einen zusätzlichen Einlass zum dritten Einlass bzw. zu dem Einlass, welcher zum Ausgleich eines Kalzium-Verlustes vorgesehen ist.

In einigen Fällen kann es vorteilhaft sein, wenn der jeweilige Einlass zur Zufuhr des Substituats dabei in Blutflussrichtung derart angeordnet ist, dass das Substituat in Blutflussrichtung vor der Gasaustauschbehandlung dem Blutstrom zuführbar ist, wobei der zugehörige Einlass insbesondere unmittelbar vor der Gasaustauschvorrichtung angeordnet ist, bezogen auf die Blutflussrichtung, um eine möglicherweise vorhandene, unerwünschte CO2-Beladung des Substituats mittels der Gasaustauschvorrichtung ausgleichen zu können.ln manchen Anwendungsfällen ist es vorteilhafter, das Substituat erst nach der Dialyse- und Adsorptionsbehandlung dem Blutstrom zuzuführen, zur Vermeidung eines Verdünnungseffektes, der die Effektivität der Adsorptionsbehandlung verringern würde (die Stärke der Adsorption ist in der Regel konzentrationsabhängig).

Wie vorstehend bereits ausgeführt worden ist, können bei einem erfindungsgemäßen System die Adsorbervorrichtung und/oder die Plasmatrenneinrichtung, die Dialysevorrichtung und die Gasaustauschvorrichtung grundsätzlich in beliebiger Reihenfolge sequenziell in Reihe geschaltet werden, wobei jedoch bestimmte Anordnungen, d.h. eine bestimmte Reihenfolge der Durchströmung der einzelnen Behandlungsvorrichtungen in Blutflussrichtung, besonders vorteilhaft sind.

Eine besonders vorteilhafte Ausgestaltung eines erfindungsgemäßen Systems zur extrakorporalen Blutbehandlung ergibt sich, wenn die Adsorbervorrichtung und/oder die Plasmatrennvorrichtung in Blutflussrichtung vor der Gasaustauschvorrichtung angeordnet ist. Da nach einer Adsorptionsbehandlung dem Blutmassenstrom in der Regel zum Ausgleich des bei der Adsorptionsbehandlung entzogenen Volumens ebenfalls ein Substituat, in der Regel eine physiologische Substitutionsflüssigkeit, vorzugsweise eine Elektrolytlösung, zuzuführen ist, welche in einigen Fällen mit CO2 beladen sein kann, ist es vorteilhaft, wenn die Adsorbervorrichtung in Blutflussrichtung vor der Gasaustauschvorrichtung angeordnet ist, da auf diese Weise eine durch die Substitutionsflüssigkeit bewirkte, unerwünschte Aufladung mit CO2 mittels der Gasaustauschvorrichtung wieder ausgeglichen werden kann. Damit kann im Ergebnis eine verbesserte CO2-Entfernung aus dem zu behandelnden Blutmassenstrom gegenüber einer Anordnung der Gasaustauschvorrichtung vor der Adsorbervorrichtung erreicht werden.

In einer alternativen, in einigen Fällen ebenfalls vorteilhaften Ausgestaltung eines erfindungsgemäßen Systems ist die Adsorbervorrichtung und/oder die Plasmatrennvorrichtung in Blutflussrichtung nach der Gasaustauschvorrichtung angeordnet. Vorteilhaft an dieser Anordnung ist, dass durch den an der Adsorbervorrichtung entstehenden Staudruck eine Erhöhung des Druckgefälles in der Gasaustauschvorrichtung erreicht werden kann, insbesondere an der Gasaustauschmembran, wodurch der Gasaustausch verbessert werden kann.

Zur Zufuhr des erforderlichen Substituts zum Ausgleich des in der Adsorbervorrichtung bei der Adsorptionsbehandlung entstandenen Volumenverlustes weist ein erfindungsgemäßes System vorzugsweise einen weiteren, insbesondere vierten, Einlass zum Blutmassenstrom auf, welcher insbesondere in Blutflussrichtung derart angeordnet ist, dass das Substituat in Blutflussrichtung nach dem Absorptionsvorgang, insbesondere nach der Behandlungsstrecke in der Adsorbervorrichtung, dem Blutmassenstrom zuführbar ist, wobei der vierte Einlass insbesondere unmittelbar nach der Adsorbervorrichtung angeordnet ist, bezogen auf die Blutflussrichtung.

In einigen Fällen kann es aber auch vorteilhaft(er) sein, den vierten Einlass vor der Adsorbervorrichtung anzuordnen, insbesondere stromaufwärts von der Gasaustauschvorrichtung, um eine durch die zum Ausgleich des bei der Adsorptionsbehandlung entstehenden Flüssigkeitsverlustes erforderliche Substitutionsflüssigkeit bewirkte, unerwünschte Aufladung des Blutstroms mit CO2 mittels der Gasaustauschvorrichtung ausgleichen zu können. Dies ist insbesondere der Fall, wenn die Adsorbervorrichtung in Blutflussrichtung nach der Gasaustauschvorrichtung angeordnet ist, d.h. stromabwärts zur Gasaustauschvorrichtung.

In einer weiteren vorteilhaften Ausgestaltung eines erfindungsgemäßen Systems ist die Adsorbervorrichtung und/oder die Plasmatrenneinrichtung in Blutflussrichtung vor der Dialysevorrichtung angeordnet, insbesondere, wenn die Dialysevorrichtung zur Hämodialyse oder Hämodiafiltration ausgebildet ist und insbesondere einen Dialysator aufweist. Die Anordnung der Adsorbervorrichtung blutstromaufwärts, d.h. in Blutflussrichtung vor der Dialysevorrichtung, hat den Vorteil, dass der Adsorbervorrichtung kein durch das Dialysat verdünnter Blutstrom zugeführt wird, wodurch eine besonders hohe Effizienz der Adsorptionsbehandlung erreicht werden kann.

Ferner können im Absorber entstehende, unspezifische lonenbindungen oder pH-Verschiebungen durch die nachfolgend angeordnete Dialysevorrichtung ausgeglichen werden.

Des Weiteren kann die blutstromabwärts angeordnete Dialysevorrichtung durch ihren Aufbau aus kleinsten Hohlfasern als Sicherheitssystem gegen einen unerwünschten Partikeleintrag aus der Adsorbervorrichtung wirken.

Ist die Dialysevorrichtung eines erfindungsgemäßen Systems zur Hämodialyse oder Hämodiafiltration ausgebildet und weist insbesondere einen Dialysator auf, weist ein erfindungsgemäßes System, insbesondere die Dialysevorrichtung, vorzugsweise einen fünften Einlass zur Zufuhr eines Dialysats, d. h. einer Dialysierflüssigkeit, auf.

Zur Abfuhr eines bei der Dialysebehandlung in der Dialysevorrichtung entstehenden Effluent, weist ein erfindungsgemäßes System vorzugsweise einen zweiten Auslass auf.

In einer alternativen Ausgestaltung eines erfindungsgemäßen Systems ist die Adsorbervorrichtung in Blutflussrichtung nach der Dialysevorrichtung angeordnet, insbesondere, wenn die Dialysevorrichtung zur Hämofiltration oder Hämodiafiltration ausgebildet ist und vorzugsweise einen Hämofilter aufweist. Diese Reihenfolge kann in einigen Anwendungsfällen vorteilhaft sein, insbesondere wenn eine besonders effektive Adsorptionsbehandlung angezeigt ist, da in diesem Fall der zu behandelnde Blutstrom durch die Hämofiltration in der Dialysevorrichtung aufkonzentriert werden kann, wodurch die Effektivität der Adsorptionsbehandlung in der nachfolgend angeordneten Adsorbervorrichtung gesteigert werden kann.

In diesem Fall steigt zwar das Clotting-Risiko des Systems, insbesondere in der Adsorbervorrichtung, gegenüber den zuvor beschriebenen Ausgestaltungen eines erfindungsgemäßen Systems. Durch entsprechende Überwachungsmaßnahmen, wie beispielsweise jeweils vor und nach wenigstens einer Blutbehandlungsvorrichtung angeordnete Drucksensoreinrichtungen, anhand derer auf den Zustand der jeweiligen Blutbehandlungsvorrichtung geschlossen werden kann, kann beginnendes Clotting in vielen Fällen relativ zuverlässig und zeitnah erkannt werden. Dadurch, insbesondere in Verbindung mit dem zusätzlichen Einsatz von Antikoangulatien, kann das Clotting-Risiko in den meisten Fällen gut beherrscht werden.

In einer weiteren vorteilhaften Ausgestaltung eines erfindungsgemäßen Systems ist die Dialysevorrichtung in Blutflussrichtung vor der Gasaustauschvorrichtung angeordnet, insbesondere, wenn die Dialysevorrichtung zur Hämodialyse oder Hämofiltration ausgebildet ist und zur Dialysebehandlung die Zufuhr eines Dialysats erfordert. In diesem Fall kann eine unerwünschte Aufladung mit CO2 durch ein unter Umständen mit CO2 beladenes Dialysat in der Dialysevorrichtung mittels der in Blutflussrichtung sequenziell nach der Dialysevorrichtung angeordnete Gasaustauschvorrichtung vor der Rückführung des behandelten Blutstroms in den intrakorporalen Blutkreislauf eines zu behandelnden Patienten oder Tieres ausgeglichen werden, was bei einer Anordnung der Dialysevorrichtung nach der Gasaustauschvorrichtung nicht der Fall wäre.

In einer alternativen, aber in einigen Fällen ebenfalls vorteilhaften Ausgestaltung eines erfindungsgemäßen Systems ist die Dialysevorrichtung in Blutflussrichtung nach der Gasaustauschvorrichtung angeordnet, wobei in diesem Fall die Dialysevorrichtung vorzugsweise zur Hämofiltration ausgebildet ist und einen Hämofilter aufweist und insbesondere nicht zur Hämodialyse ausgebildet ist.

Durch den der Gasaustauschvorrichtung nachgeschalteten Hämofilter kann das Druckgefälle zwischen Blutseite und Gasseite an der Gasaustauschvorrichtung erhöht werden, wodurch die Effizienz der Gasaustauschvorrichtung verbessert werden kann.

Ist die Dialysevorrichtung lediglich zur Hämofiltration ausgebildet und nicht zur Hämodialyse insbesondere auch nicht zur Hämodiafiltration, tritt der Nachteil einer unerwünschten CO2-Aufladung infolge eines Austausch des Blutstroms mit einem zugeführten, unter Umständen mit CO2 beladenen Dialysats wie bei der Hämodialyse oder der Hämodiafiltration möglich, nicht auf. In letzterem Fall kann die Substitutionslösung zum Volumenausgleich vorzugweise bereits stromaufwärts des Gasaustauschers zugegeben werden, um die mögliche CO2-Beladung mittels der Gasaustauschvorrichtung zumindest teilweise, vorzugsweise vollständig, ausgleichen zu können.

Bevorzugt weist ein erfindungsgemäßes System, insbesondere die Gasaustauschvorrichtung einen dritten Auslass zur Abfuhr von aus dem behandelten Blutstrom entfernten Kohlendioxid (CO2) auf und/oder einen weiteren, insbesondere sechsten, Einlass zur Zufuhr eines Gases oder Gasgemisches zur Anreicherung des zu behandelnden Blutstroms, insbesondere zur Zufuhr von Sauerstoff (02) und/oder eines medizinischen Gasgemisches und/oder zur Zufuhr eines Spülgases, wie beispielsweise Sauerstoff (02), ein Gasgemisch und/oder Umgebungsluft.

In einer weiteren vorteilhaften Ausgestaltung eines erfindungsgemäßen Systems weist das System wenigstens eine Drucksensoreinrichtung zur Ermittlung eines Strömungsdruckes des Blutstroms an wenigstens einer definierten Stelle im System auf, wobei vorzugsweise in Blutflussrichtung unmittelbar vor und/oder unmittelbar nach wenigstens einer Behandlungsstrecke einer Blutbehandlungsvorrichtung wenigstens eine Drucksensoreinrichtung angeordnet ist.

Sind vor und nach wenigstens einer Behandlungsstrecke jeweils Drucksensoreinrichtungen vorgesehen, kann auf diese Weise der Druckabfall über die Behandlungsstrecke erfasst werden, von welchem auf den Zustand der zugehörigen Blutbehandlungsvorrichtung geschlossen werden kann. Insbesondere lässt sich auf diese Weise beurteilen, inwiefern eine Blutbehandlungsvorrichtung von Clotting betroffen ist, wobei ein plötzlich stark ansteigender Druckabfall einen Hinweis darauf gibt, dass die jeweilige Blutbehandlungsvorrichtung von Clotting betroffen ist.

In einer bevorzugten Ausgestaltung weist ein erfindungsgemäßes System eine Steuerungseinrichtung auf, wobei beim erfindungsgemäßen System die Steuerungseinrichtung insbesondere zur Steuerung und/oder Regelung sämtlicher steuerbaren und/oder regelbaren Komponenten des Systems ausgebildet ist. D.h. mit anderen Worten weist ein erfindungsgemäßes System in einer bevorzugten Ausgestaltung eine gemeinsame Steuerung zur Steuerung sämtlicher Blutbehandlungsvorrichtungen auf. Die Steuerung ist dabei insbesondere zur Steuerung einer oder mehrerer Pumpen, und/oder zur Steuerung von Zufluss- und/oder Abflussmengen von Stoffen und/oder Zusammensetzungen und/oder zur Auswertung der von wenigstens einer Sensoreinrichtung erfassten Sensordaten und/oder zur Überwachung des erfindungsgemäßen Systems ausgebildet, insbesondere zur Steuerung und/oder Regelung des zu behandelnden Blutstroms.

Bevorzugt können mithilfe einer oder mehrerer Drucksensoreinrichtungen Blockaden oder eine Unterbrechung des Blutkreislaufs erkannt werden und entsprechende Maßnahmen, wie beispielsweise die Auslösung eines Alarms oder die Abschaltung des Systems, insbesondere eine den Blutstrom fördernde Pumpe, abgeschaltet werden.

Vorzugsweise ist ein erfindungsgemäßes System dabei derart ausgebildet, dass ein für eine möglichst hohe Effektivität einer Blutbehandlung erforderlicher, definierter Transmembrandruck für wenigstens eine der Blutbehandlungsvorrichtungen, insbesondere für die Dialysevorrichtung, bezogen auf einen Grenzwert überwacht wird. Vorzugsweise kann in Abhängigkeit wenigstens eines von einer Drucksensoreinrichtung erfassten Sensorsignals der Fluss des Blutstroms derart eingestellt werden, dass sich ein gewünschter definierter Transmembrandruck einstellt bzw. ergibt, so dass eine verbesserte Blutbehandlung erreicht werden kann.

In einer weiteren vorteilhaften Ausgestaltung eines erfindungsgemäßen Systems weist ein erfindungsgemäßes System vorzugsweise wenigstens eine Gasblasen-Detektionseinrichtung zum Erkennen einer Gasblase im Blutstrom auf. Vorzugsweise ist das System dabei derart ausgebildet, dass, wenn mittels der Gasblasen-Detektionseinrichtung eine Gasblase erkannt worden ist, ein insbesondere vor dem ersten Auslass, vorzugsweise in einer Rückführleitung, angeordnetes Absperrventil geschlossen werden kann, um eine Rückführung der Gasblase mit dem behandelten Blutstrom in den intrakorporalen Kreislauf, insbesondere den intrakorporalen Blutkreislauf, eines zu behandelnden Patienten oder eines zu behandelnden Tieres zu verhindern. Ferner können vorzugsweise zusätzlich sämtliche Pumpen, die zur Förderung des Blutstroms dienen, abgeschaltet werden.

In einer weiteren vorteilhaften Ausgestaltung eines erfindungsgemäßen Systems ist bei wenigstens einer Blutbehandlungsvorrichtung eine Behandlungsstrecke zumindest teilweise, vorzugweise vollständig, durch ein austauschbares Behandlungsmodul gebildet, insbesondere durch ein kartuschenartiges Behandlungsmodul. Eine derartige Ausgestaltung einer Blutbehandlungsvorrichtung ermöglicht einen flexiblen Austausch des jeweiligen Behandlungsmoduls, insbesondere eine einfache und flexible Anpassung der einzelnen Behandlungsvorrichtungen an die jeweils erforderliche Blutbehandlung.

Beispielsweise kann auf diese Weise leicht ein Endotoxin-Adsorber-Behandlungsmodul gegen ein Zytokin-Adsorber-Behandlungsmodul mit einer anderen funktionalen Adsorptionsschicht ausgetauscht werden oder ein spezielles Immun-Adsorber-Behandlungsmodul eingesetzt werden oder ein Hämofilter gegen einen Dialysator getauscht werden oder dergleichen. Dadurch erhöht sich die Bandbreite der mit einem erfindungsgemäßen System möglichen Behandlungen erheblich, wodurch die Wirtschaftlichkeit eines erfindungsgemäßen Systems deutlich gesteigert werden kann.

In einer weiteren vorteilhaften Ausgestaltung eines erfindungsgemäßen Systems weist das System wenigstens eine schaltbare Bypasseinrichtung zur Umgehung wenigstens einer Blutbehandlungsvorrichtung auf. Dadurch kann bei Bedarf ein erfindungsgemäßes System auch für Blutbehandlungen eingesetzt werden, die jeweils nur die Verwendung einer oder zweier der drei Blutbehandlungsvorrichtungen des Systems erfordern, jedoch nicht die Durchströmung durch sämtliche drei Blutbehandlungsvorrichtungen eines erfindungsgemäßen Systems erforderlich machen. Auf diese Weise kann beispielsweise eine erforderliche Blutbehandlung, welche nur eine Adsorptionsbehandlung und/oder nur eine Plasmatrennung und eine Dialysebehandlung erfordert unter Umgehung der Gasaustauschvorrichtung erfolgen. Ebenso ist eine Dialysebehandlung mit nachfolgendem Gasaustausch, insbesondere nachfolgender CO2-Entfernung, ohne eine gleichzeitige Adsorptionsbehandlung möglich. Dadurch kann die Bandbreite an Behandlungsmöglichkeiten eines erfindungsgemäßen Systems deutlich vergrößert werden. Ferner können die Behandlungskosten in einer Vielzahl an Behandlungsfällen deutlich reduziert werden, da der Materialverbrauch deutlich gesenkt werden kann, weil nicht jeweils drei Behandlungsmodule verbraucht werden. Darüber hinaus kann eine Behandlungsvorrichtung aus dem System entfernt werden, wenn diese ihre Funktion nicht mehr erfüllt, weil sie beispielsweise geclottet oder erschöpft ist, wie beispielsweise ein Adsorber, der vollständig beladen ist.

Vorzugsweise weist wenigstens eine Bypasseinrichtung wenigstens ein Bypassventil sowie eine zugehörige Bypassleitung auf, die insbesondere fluidisch mit einer Hauptleitung verbunden oder verbindbar ist, wobei insbesondere mittels eines Bypassventils die zugehörige Bypassleitung geöffnet oder verschlossen werden kann, sodass ein zu behandelnder Blutstrom gezielt entlang der zugehörigen Bypassleitung geführt werden kann oder gezielt durch die nachfolgende Blutbehandlungsvorrichtung bzw. die nachfolgende Blutbehandlungsstrecke geleitet werden kann.

Bevorzugt ist wenigstens ein Bypassventil dabei derart ausgebildet, dass bei geöffnetem Bypassventil keine Durchströmung der nachgelagerten Behandlungsstrecke erfolgt, d.h. dass die nachfolgende Blutbehandlungsvorrichtung vorzugsweise vollständig verschlossen werden kann und der gesamte zu behandelnden Blutstrom über die zugehörige Bypassleitung an der zugehörigen Blutbehandlungsvorrichtung, vorbeigeführt werden kann, d.h. die Blutbehandlungsvorrichtung umgangen werden kann.

Unter "Umgehen einer Blutbehandlungsvorrichtung" wird im Sinne der vorliegenden Erfindung insbesondere verstanden, die Hauptleitung zu verzweigen, insbesondere an einer Verzweigung stromaufwärts der Blutbehandlungsvorrichtung oder in der Blutbehandlungsvorrichtung, eine Bypassleitung um die Blutbehandlungsvorrichtung herumzuführen und die Bypassleitung stromabwärts wieder mit der Hauptleitung zusammenzuführen, insbesondere nach der Blutbehandlungsvorrichtung oder in der Blutbehandlungsvorrichtung

Bei geschlossenem Bypassventil hingegen ist vorzugsweise die Bypassleitung verschlossen, insbesondere vollständig, sodass der gesamte behandelnde Blutstrom durch die nachfolgende Behandlungsstrecke bzw. die nachfolgende Blutbehandlungsvorrichtung strömt.

Auf diese Weise können gezielt nur die erforderlichen Behandlungsvorrichtungen durchströmt werden. Dadurch können die Einsatzmöglichkeiten eines erfindungsgemäßen Systems deutlich gesteigert werden. Insbesondere kann auf diese Weise die Auslastung bzw. die Nutzungszeit eines erfindungsgemäßen Systems verbessert werden, wodurch wiederum Wirtschaftlichkeit gesteigert werden kann.

Zur Einstellung einer definierten Flussrate des Blutstroms an wenigstens einer der Blutbehandlungsvorrichtungen, insbesondere zum Erzeugen eines gewünschten, definierten Transmembrandrucks, insbesondere für wenigstens eine zugehörige Behandlungsstrecke, kann ein erfindungsgemäßes System wenigstens eine, insbesondere in einem Abschnitt zwischen einer Verzweigung und der anschließenden Zusammenführung in der Hauptleitung und/oder der Bypassleitung angeordnete weitere Pumpe aufweisen.

Bei entsprechender Anzahl und Ausgestaltung der einzelnen Pumpen kann auf diese Art und Weise eine unabhängige Einstellung der Blutflussraten in den einzelnen Behandlungsvorrichtungen erreicht werden, wobei das System dazu insbesondere eine oder mehrere entsprechend ausgebildete Steuerungseinrichtungen aufweist.

In einer vorteilhaften Ausgestaltung ist wenigstens eine einer Blutbehandlungsvorrichtung zugeordnete Bypasseinrichtung derart ausgebildet, dass ein rezirkulierender Blutfluss über und/oder durch die zugehörige Blutbehandlungsvorrichtung bewirkbar ist.

In einer weiteren vorteilhaften Ausgestaltung eines erfindungsgemäßen Systems ist in der zugehörigen Bypassleitung wenigstens einer Blutbehandlungsvorrichtung wenigstens eine weitere Blutbehandlungsvorrichtung angeordnet, insbesondere derart, dass ein rezirkulierender Blutfluss durch die zugehörige, in der Hauptleitung angeordnete Blutbehandlungsvorrichtung und/oder durch die weitere, in der Bypassleitung angeordnete Blutbehandlungsvorrichtung entsteht.

In einer alternativen, vorteilhaften Ausgestaltung eines erfindungsgemäßen Systems ist die erste Blutbehandlungsvorrichtung eine Plasmatrenneinrichtung, welche vorzugsweise mittels einer Bypassleitung umgehbar ist, wobei insbesondere in der Bypassleitung eine weitere Blutbehandlungsvorrichtung in Form einer Adsorbervorrichtung angeordnet ist. Dabei ist die Adsorbervorrichtung bevorzugt stromabwärts einer in der Bypassleitung angeordneten Pumpe angeordnet.

Das aus der Absorbervorrichtung abgeführte behandelte Blut bzw. das aus der Adsorbervorrichtung abgeführte, behandelte Blutplasma, kann dabei entweder nach der Plasmatrenneinrichtung der Hauptleitung zugeführt werden oder rezirkulierend der Plasmatrenneinrichtung zugeführt werden.

In einer alternativen Ausgestaltung eines erfindungsgemäßen Systems kann abgetrenntes Plasma aus der Plasmatrennvorrichtung, insbesondere mithilfe einer Pumpe, abgeführt und einem Plasma-Entsorgungsbehälter zugeführt werden und mittels eines weiteren Einlasses, insbesondere der Hauptleitung, vorzugsweise mithilfe einer weiteren Pumpe, frisches Plasma, insbesondere aus einer Speichervorrichtung, zugeführt werden.

In einer weiteren vorteilhaften Ausgestaltung eines erfindungsgemäßen Systems weist wenigstens eine Komponente des Systems an einer mit dem zu behandelnden Blutstrom in Kontakt kommenden Oberfläche eine biokompatible und vorzugsweise funktionale Beschichtung auf, insbesondere eine antibakterielle, gerinnungshemmende und/oder anti-inflammatorische Beschichtung. Vorzugsweise ist wenigstens ein Lumen des Systems, welches zur Durchströmung mit einem zu behandelnden und/oder behandelten Blutstroms ausgebildet ist, mit einer biokompatiblen und vorzugweise funktionalen Beschichtung versehen, insbesondere mit einer antibakteriellen, gerinnungshemmenden und/oder anti-inflammatorischen Beschichtung.

Vorzugsweise weist mindestens eine Oberfläche des erfindungsgemäßen Systems dabei eine heparinhaltige und/oder albumin- und heparinhaltige Beschichtung auf. In einigen Fällen kann es vorteilhaft sein, wenn das System hingegen lediglich heparinfreie Beschichtungen aufweist, denn dadurch kann das System auch zur Behandlung von Patienten oder Tieren mit einer Heparinunverträglichkeit eingesetzt werden.

In einer alternativen und/oder zusätzlichen bevorzugten Ausgestaltung eines erfindungsgemäßen Systems weist wenigstens eine Beschichtung definierte Antikörper und/oder ein oder mehrere Enzyme auf. Auch eine antibakterielle Beschichtung ist denkbar.

In einer weiteren vorteilhaften Ausgestaltung eines erfindungsgemäßen Systems ist wenigstens eine Schutzschicht zum Schutz der funktionalen Beschichtung aufgebracht, wobei die Schutzschicht vorzugsweise dazu dient, eine Sterilisation und/oder Lagerung einzelner, beschichteter Komponenten des Systems ohne einen nennenswerten Verlust der Funktionalität der funktionalen Beschichtung zu ermöglichen.

In einer besonders bevorzugten Ausgestaltung eines erfindungsgemäßen Systems weist das System wenigstens eine Oberfläche mit einer mittels der SPSO-Technologie von der Leukocare AG ausgebildeten Beschichtung auf.

Eine erfindungsgemäße Behandlungsvorrichtung zur extrakorporalen Blutbehandlung, weist ein gemäß der vorliegenden Erfindung ausgebildetes System zur extrakorporalen Blutbehandlung auf, wobei die erste, die zweite und die dritte Blutbehandlungsvorrichtung des Systems insbesondere in einem gemeinsamen Gehäuse angeordnet sind und/oder von einer gemeinsamen Basis, d.h. von einer gemeinsamen Trägereinrichtung, aufgenommen sind.

Vorzugsweise sind die drei Blutbehandlungsvorrichtungen dabei in einem gemeinsamen Gehäuse angeordnet und/oder sind von einer gemeinsamen Basis, wie beispielsweise einer gemeinsamen Trägervorrichtung oder dergleichen, aufgenommen. Besonders bevorzugt sind die einzelnen Blutbehandlungsvorrichtungen dabei jeweils austauschbar in und/oder an dem gemeinsamen Gehäuse und/oder der Basis befestigt, insbesondere jeweils als austauschbare Module. Dadurch lässt sich ein besonders kompaktes, erfindungsgemäßes System bereitstellen, das gleichzeitig flexibel auf den jeweiligen Behandlungsfall gezielt abgestimmt und ausgestaltet werden kann.

Ein Kit und/oder Set zur extrakorporalen Blutbehandlung weist als Komponenten wenigstens eine erste Blutbehandlungsvorrichtung, eine zweite Blutbehandlungsvorrichtung, eine dritte Blutbehandlungsvorrichtung und ein Schlauchset mit einem ersten Einlass zum Einbringen eines zu behandelnden Blutstroms und einem ersten Auslass zum Ausbringen eines behandelten Blutstroms mit einem oder mehreren Schläuchen auf sowie insbesondere eine Installations- und/oder Bedienungsanleitung.

Dabei ist die erste Blutbehandlungsvorrichtung eine Adsorbervorrichtung zur Entfernung wenigstens eines körperfremden und/oder wenigstens eines körpereigenen Pathogens und/oder eine Plasmatrennvorrichtung zum Trennen von Blutplasma von den übrigen Blutbestandteilen oder weist eine entsprechende Adsorbervorrichtung und/oder Plasmatrennvorrichtung auf. Die zweite Blutbehandlungsvorrichtung ist als Dialysevorrichtung ausgebildet, insbesondere als Dialysevorrichtung zur Nierenersatztherapie, und die dritte Blutbehandlungsvorrichtung ist als Gasaustauschvorrichtung ausgebildet, insbesondere als Gasaustauschvorrichtung zur zumindest teilweisen CO2-Entfernung aus einem die Gasaustauschvorrichtung durchströmenden Blutstrom und/oder zur Zufuhr eines Gases oder Gasgemisches in einen die Gastaustauschvorrichtung durchströmenden Blutstrom. Die Komponenten des Kits können zu einem gemäß der vorliegenden Erfindung ausgebildeten System zur extrakorporalen Blutbehandlung verbunden werden, insbesondere gemäß der Installations- und/oder Bedienungsanleitung.

Dadurch kann auf einfache Art und Weise ein besonders flexibles, erfindungsgemäßes System bereitgestellt werden, das eine gezielt auf den jeweiligen Behandlungsfall abgestimmte Kombination einer Adsorbervorrichtung und/oder einer Plasmatrenneinrichtung, einer Dialysevorrichtung und einer Gasaustauschvorrichtung ermöglicht.

D.h., ein erfindungsgemäßes System kann sowohl in Form einer gemeinsamen Behandlungsvorrichtung bereitgestellt werden, bei welcher die drei Blutbehandlungsvorrichtungen des Systems, d.h. die Adsorbervorrichtung und/oder die Plasmatrenneinrichtung, die Dialysevorrichtung und die Gasaustauschvorrichtung Teil einer gemeinsamen Vorrichtung sind, als auch in Form eines Kits und/oder Sets, bei welchem wenigstens zwei der drei Blutbehandlungsvorrichtungen separate Vorrichtungen sind, welche jedoch insbesondere mithilfe eines geeigneten Schlauchsystems mit einer oder mehreren Schlauchleitungen zu einem erfindungsgemäßen System verbunden werden können und erfindungsgemäß sequenziell derart in Reihe geschaltet werden können, dass die einzelnen Blutbehandlungsvorrichtungen jeweils nacheinander in Reihe sequenziell durchströmt werden können.

Ein Verfahren zum Betreiben eines erfindungsgemäßen Systems zur extrakorporalen Blutbehandlung und/oder einer erfindungsgemäßen Behandlungsvorrichtung ist gekennzeichnet durch die Schritte:
- Bereitstellen einer zu behandelnden Blutmenge,
- Einbringen eines zu behandelnden Blutstroms in das System über den ersten Einlass des Systems,
- Durchströmen wenigstens einer der Blutbehandlungsvorrichtungen und
- Ausbringen des behandelten Blutstroms über den ersten Auslass des Systems.

Vorzugsweise werden dabei, sofern das zur extrakorporalen Blutbehandlung verwendete System dazu ausgebildet ist und wenigstens eine insbesondere schaltbare Bypasseinrichtung aufweist, je nach Bedarf nur diejenigen Blutbehandlungsvorrichtungen durchströmt, deren Nutzung zur jeweiligen, erforderlichen Behandlung angezeigt ist.

In einer vorteilhaften Ausgestaltung eines Verfahrens zum Betreiben eines erfindungsgemäßen Systems wird die zu behandelnde Blutmasse dabei in einem Behältnis bereitgestellt, insbesondere in einem Behälter oder einem Beutel, wobei das behandelte Blut vorzugsweise in ein Behältnis, insbesondere in einen Behälter oder in einen Beutel, ausgebracht wird. Alternativ kann das behandelte Blut auch einem separierten, zur Transplantation vorgesehenen Organ zugeführt werden oder einem zu behandelnden Patienten oder einem zu behandelnden Tier.

Ein Verfahren zur extrakorporalen Blutbehandlung mit einem erfindungsgemäßen System oder einer erfindungsgemäßen Behandlungsvorrichtung ist gekennzeichnet durch die Schritte:
- Einbringen des Systems in einen Blutkreislauf eines zu behandelnden Menschen oder Tieres und Herstellen eines extrakorporalen Blutkreislaufs durch Verbinden des ersten Einlasses des Systems mit einem ersten Blutgefäß des zu behandelnden Menschen oder Tieres und Verbinden des ersten Auslasses des Systems mit dem ersten Blutgefäß und/oder einem zweiten Blutgefäß des Menschen oder Tieres,
- Entnehmen eines zu behandelnden Blutstroms aus dem intrakorporalen Blutkreislauf des Menschen oder Tieres und Einbringen des zu behandelnden Blutstroms in das System über den ersten Einlass des Systems,
- Durchströmen wenigstens einer der Blutbehandlungsvorrichtungen,
- Ausbringen des behandelten Blutstroms über den ersten Auslass des Systems und Rückführen des behandelten Blutstroms in den intrakorporalen Blutkreislauf des Menschen oder Tieres.

Das erfindungsgemäße System kann dazu veno-venös oder arterio-venös mit dem intrakorporalen Blutkreislauf eines zu behandelnden Menschen oder Tieres verbunden werden oder alternativ über wenigstens einen künstlich angelegten Blutzugang, je nach Bedarf.

Zum veno-venösen Verbinden eines erfindungsgemäßen Systems mit einem intrakorporalen Blutkreislauf eines zu behandelnden Menschen oder Tieres eignet sich insbesondere eine doppellumige Kanüle mit konzentrisch zueinander angeordnetem Einlass und Auslass, bei erwachsenen Patienten beispielsweise die unter der Bezeichnung "Nova-Port^{®} twin" von der Firma Novalung GmbH vertriebene Kanüle. D.h., bei einem erfindungsgemäßen Verfahren zur extrakorporalen Blutbehandlung erfolgt das Einbringen des Systems in einen Blutkreislauf eines zu behandelnden Menschen oder Tieres und das Herstellen des extrakorporalen Blutkreislaufs vorzugsweise unter Verwendung einer doppellumigen Kanüle.

Diese und weitere Merkmale der Erfindung gehen außer aus den Ansprüchen und aus der Beschreibung auch aus den zugehörigen Figuren sowie der Figurenbeschreibung hervor, wobei sämtliche der genannten und/oder dargestellten Merkmale und Merkmalskombinationen nicht nur in der jeweils angegebenen Kombination, sondern auch in anderen Kombinationen oder in Alleinstellung bei einer Ausgestaltung der Erfindung verwirklicht sein können, sofern dies technisch sinnvoll ist.

Manche der genannten und/oder dargestellten Merkmale bzw. Eigenschaften der Erfindung betreffen sowohl ein erfindungsgemäßes System und eine erfindungsgemäße Behandlungsvorrichtung, wobei einige dieser Merkmale und Eigenschaften nur einmal beschrieben sind, beispielsweise nur im Zusammenhang mit dem erfindungsgemäßen System, jedoch unabhängig davon im Rahmen technisch möglicher Ausgestaltungen sowohl für ein erfindungsgemäßes System, als auch für eine erfindungsgemäße Behandlungsvorrichtung gelten.

Im Folgenden wird die Erfindung anhand mehrerer Ausführungsbeispiele unter Bezugnahme auf die beigefügten Figuren näher erläutert, wobei funktionsgleiche Bauteile mit gleichen Bezugszeichen bezeichnet sind, sofern keine expliziten anderslautenden Angaben gemacht sind oder sich aus dem Kontext nichts anderes ergibt. Es zeigen:
- Fig. 1: in schematischer Darstellung den grundsätzlichen Aufbau eines ersten Ausführungsbeispiels eines erfindungsgemäßen Systems,
- Fig. 2: in schematischer Darstellung den grundsätzlichen Aufbau eines zweiten Ausführungsbeispiels eines erfindungsgemäßen Systems,
- Fig. 3: in schematischer Darstellung den grundsätzlichen Aufbau eines dritten Ausführungsbeispiels eines erfindungsgemäßen Systems,
- Fig. 4: in schematischer Darstellung den grundsätzlichen Aufbau eines vierten Ausführungsbeispiels eines erfindungsgemäßen Systems,
- Fig. 5: in schematischer Darstellung den grundsätzlichen Aufbau eines fünften Ausführungsbeispiels eines erfindungsgemäßen Systems,
- Fig. 6: in schematischer Darstellung den grundsätzlichen Aufbau eines sechsten Ausführungsbeispiels eines erfindungsgemäßen Systems,
- Fig. 7: in schematischer Darstellung den grundsätzlichen Aufbau eines siebten Ausführungsbeispiels eines erfindungsgemäßen Systems,
- Fig. 8: in schematischer Darstellung den grundsätzlichen Aufbau eines achten Ausführungsbeispiels eines erfindungsgemäßen Systems,
- Fig. 9: in schematischer Darstellung den grundsätzlichen Aufbau eines neunten Ausführungsbeispiels eines erfindungsgemäßen Systems und
- Fig. 10: in schematischer Darstellung den grundsätzlichen Aufbau eines zehnten Ausführungsbeispiels eines erfindungsgemäßen Systems.

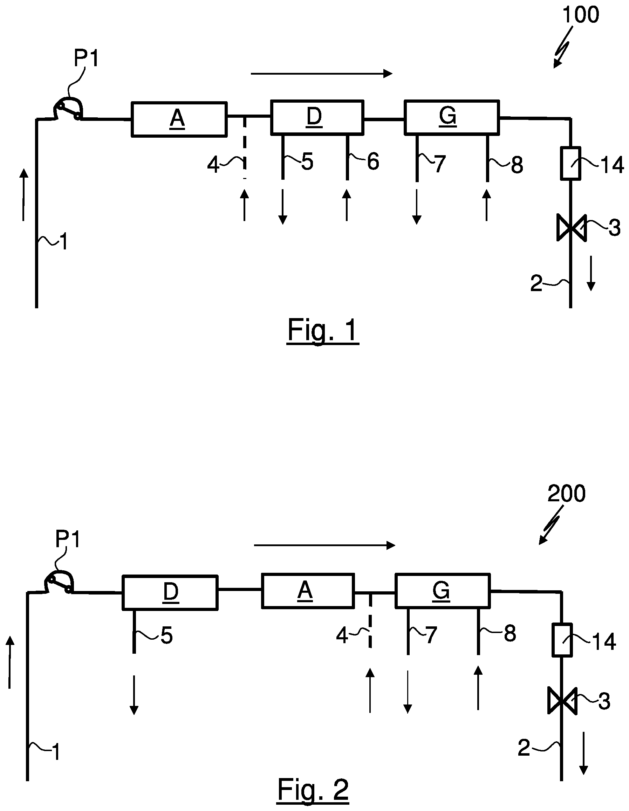

Fig. 1 zeigt in schematischer Darstellung den grundsätzlichen Aufbau eines ersten Ausführungsbeispiels eines erfindungsgemäßen Systems 100 zur extrakorporalen Blutbehandlung, wobei das System 100 einen durch eine Zuführleitung 1 gebildeten ersten Einlass zum Einbringen eines zu behandelnden Blutstroms in das System 100, drei Blutbehandlungsvorrichtungen A, D und G sowie einen durch eine Rückführleitung 2 gebildeten ersten Auslass zum Ausbringen eines behandelten Blutstroms aus dem System 100 aufweist.

Eine erste Blutbehandlungsvorrichtung A ist dabei eine zur Endotoxin-Adsorption ausgebildete Adsorbervorrichtung A. Eine zweite Blutbehandlungsvorrichtung D ist als Dialysevorrichtung D ausgebildet, insbesondere zur Hämodialyse. Eine dritte Blutbehandlungsvorrichtung G ist ein Oxygenator zur Entfernung von Kohlendioxid (CO2) aus dem zu behandelnden Blutstrom sowie zur Anreicherung des zu behandelnden Blutstroms mit Sauerstoff (02).

Das in Fig. 1 dargestellte erfindungsgemäße System 100 ist dabei dazu ausgebildet in einen menschlichen oder tierischen Blutkreislauf zur Herstellung eines extrakorporalen Blutkreislaufs eingebracht zu werden, wobei dazu die Zuführleitung 1 mit einem ersten Blutgefäß des zu behandelnden Patienten bzw. eines zu behandelnden Tieres, insbesondere einer Vene oder einer Arterie, verbunden werden kann zur Entnahme eines zu behandelnden Blutstroms und die Rückführleitung 2 mit dem ersten Blutgefäß oder einem zweiten Blutgefäß, insbesondere einer Vene, zur Rückführung eines behandelten Blutstroms in den intrakorporalen Blutkreislauf des Patienten bzw. des Tieres.

Zur Herstellung des extrakorporalen Blutkreislaufs, insbesondere zur Verbindung mit dem intrakorporalen Blutkreislauf des zu behandelnden Patienten bzw. des zu behandelnden Tieres kann das System vorzugsweise mit einer doppellumigen Kanüle verbunden werden, welche mit nur einem Gefäßzugang die Herstellung eines veno-venösen, extrakorporalen Blutkreislaufs ermöglicht. Dadurch ist die Belastung für den zu behandelnden Patienten bzw. das zu behandelnde Tier äußerst gering, da nicht zwei separate Zugänge zu zwei separaten Gefäßen gelegt werden müssen. Ferner reduziert sich die Infektionsgefahr.

Erfindungsgemäß sind die drei Blutbehandlungsvorrichtung A, D und G dabei in Reihe, d.h. sequenziell geschaltet bezogen auf eine Blutflussrichtung eines das System 100 durchströmenden Blutstroms welcher durch die Pfeile in Fig. 1 symbolisiert dargestellt ist. Die einzelnen Blutbehandlungsvorrichtungen A, D und G sind dabei Teil einer gemeinsamen, erfindungsgemäßen Behandlungsvorrichtung und von einer gemeinsamen Basis aufgenommen, insbesondere an einem gemeinsamen Träger befestigt, wobei die einzelnen Komponenten des Systems 100 durch entsprechende Schlauchleitungen derart miteinander verbunden sind, dass sie sequenziell, d.h. nacheinander durchströmt werden können.

Die erfindungsgemäße Hintereinanderschaltung einer Adsorbervorrichtung A, einer Dialysevorrichtung D sowie einer Gasaustauschvorrichtung G ermöglicht eine kombinierte Blutbehandlung, insbesondere die Kombination einer Adsorptionstherapie, im vorliegenden Fall die Kombination einer Sepsis-Therapie mit einer Dialyse-Therapie sowie der CO2-Entfernung aus dem Blut und/oder einer Anreicherung des Blutes mit Sauerstoff (02) in einem einzigen, gemeinsamen extrakorporalen Blutkreislauf. Dadurch kann vermieden werden, dass zur Blutbehandlung mehrere extrakorporale Kreisläufe hergestellt werden müssen und entsprechend mehrere Zugänge bei einem Patienten oder zu behandelnden Tier gelegt werden müssen. Somit ermöglicht ein erfindungsgemäßes System 100 die gleichzeitige Blutbehandlung mittels Adsorption, Dialyse und Gasaustausch mit dem Blutvolumen nur eines extrakorporalen Blutkreislaufs.

Die Adsorbervorrichtung A ist bei diesem Ausführungsbeispiel eines erfindungsgemäßen Systems 100 zur Sepsis-Therapie ausgebildet. Adsorbervorrichtungen als solche zu diesem Zweck sind aus dem Stand der Technik grundsätzlich bekannt. Da dem Blutmassenstrom bei der Blutbehandlung mittels der Adsorbervorrichtung A Volumenanteile entzogen werden, ist in Blutflussrichtung nach der Adsorbervorrichtung A ein weiterer, insbesondere vierter, Einlass 4, zur Zufuhr eines Substituats nachgeschaltet, um diesen Volumenverlust auszugleichen, wobei insbesondere ein flüssiges Substituat, insbesondere eine Elektrolytlösung, zugeführt werden kann.

Bei dem in Fig. 1 dargestellten erfindungsgemäßen System 100 weist die Dialysevorrichtung D einen Dialysator zur Hämodialyse auf, dem über einem fünften Einlass 6 ein Dialysat bzw. eine Dialysierflüssigkeit zugeführt werden kann und von dem über einen zweiten Auslass 5 ein bei der Dialysebehandlung entstehendes Effluent abgeführt werden kann. Dialysevorrichtungen dieser Art sind aus dem Stand der Technik ebenfalls grundsätzlich bekannt.

Die Gasaustauschvorrichtung G ist bei dem in Fig. 1 abgebildeten, erfindungsgemäßen System 100 als Oxygenator G ausgebildet, wobei mit Hilfe des Oxygenators G aus dem hindurchströmenden Blutstrom mithilfe eines definierten Gases, welches dem Oxygenator über einen weiteren, insbesondere sechsten, Einlass 8 zugeführt werden kann, Kohlendioxid (CO2) entfernt werden, welches über einen dritten Auslass 7 abgeführt werden kann. Der durch den Oxygenator G strömende Blutstrom kann dabei außerdem mit dem definierten Gas, welches dem Oxygenator über den sechsten Einlass 8 zugeführt werden kann, und welches in diesem Fall Sauerstoff (02) ist, angereichert werden. Oxygenatoren dieser Art sind aus dem Stand der Technik ebenfalls grundsätzlich bekannt. Der Oxygenator G kann beispielsweise ein iLA^{®} Membrane Ventilator IL-1000-01 der Novalung GmbH sein.

Zur Förderung des zu behandelnden Blutstroms durch das System 100 ist eine erste, als Blutpumpe ausgebildete Pumpe P1 vorgesehen, wobei die erste Blutpumpe P1 mittels einer hier nicht dargestellten Steuerungseinrichtung, die ebenfalls Teil des erfindungsgemäßen Systems 100 ist, zur Steuerung und/oder Regelung des Blutstroms angesteuert werden kann.

Die erste Blutpumpe P1 kann dabei eine Schlauchrollenpumpe sein. In einer besonders bevorzugten Ausgestaltung weist das erfindungsgemäße System als erste Pumpe eine Zentrifugalpumpe auf. Diese kann eine als Rotorpumpe ausgebildete Diagonalpumpe sein, vorzugsweise wie in der DE 10 2010 024 650 A1 beschrieben. Die Größe der Blutpumpe P1, insbesondere deren Anschlussquerschnitte, wird je nach Blutvolumen des Patienten bzw. des zu behandelnden Tieres, ausgewählt.

Um für einen optimalen Behandlungserfolg an wenigstens einer der drei Blutbehandlungsvorrichtungen A, D und/oder G einen optimalen Blutstrom einstellen zu können sowie zu Überwachungszwecken weist das System mehrere, hier nicht dargestellte Drucksensoreinrichtungen auf, wobei bei diesem Ausführungsbeispiel eines erfindungsgemäßen Systems 100 jeweils unmittelbar vor und unmittelbar nach einer Blutbehandlungsvorrichtung A, D oder G eine entsprechende Drucksensoreinrichtung angeordnet ist.

Dies ermöglicht jeweils die Bestimmung eines über die zugehörige Blutbehandlungsvorrichtung A, D bzw. G entstehenden Druckgefälles. Mit dessen Hilfe kann auf den Zustand der jeweiligen Blutbehandlungsvorrichtung A, D bzw. G geschlossen werden. Insbesondere erlaubt der ermittelte Druckgradient einen Rückschluss darauf, inwiefern die jeweilige Blutbehandlungsvorrichtung A, D oder G von Clotting betroffen ist.

Ferner kann auf diese Weise ein in der jeweiligen Blutbehandlungsvorrichtung A, D und/oder G anliegender Transmembrandruck bestimmt werden. Da die Effizienz der jeweiligen Blutbehandlung grundsätzlich vom jeweils anliegenden Transmembrandruck abhängt und dieser für einen optimalen Behandlungserfolg in Abhängigkeit von der jeweiligen Blutbehandlungsvorrichtung innerhalb eines bestimmten Bereichs liegen sollte, lässt sich auf diese Weise, insbesondere durch eine entsprechende Ansteuerung der Blutpumpe P1, zumindest für wenigstens eine der drei Blutbehandlungsvorrichtungen A, D und G, der Blutstrom derart einstellen, dass sich jeweils ein vorteilhafter Transmembrandruck einstellt.

Das in Fig. 1 dargestellte erfindungsgemäße System 100 weist ferner eine den drei Blutbehandlungsvorrichtungen A, D und G in Blutflussrichtung nachgeschaltet angeordnete Gasblasen-Detektionseinrichtung 14 zum Erkennen einer Gasblase im Blutstrom auf sowie ein in der Rückführleitung 2, der Gasblasen-Detektionseinrichtung 14 nachgeschaltetes Absperrventil 3 auf. Wird eine Gasblase im Blutstrom mittels der Gasblasen-Detektionseinrichtung 14 detektiert, wird das Absperrventil 3 geschlossen, die erste Blutpumpe P1 abgeschaltet und ein Alarm ausgelöst. Dadurch kann verhindert werden, dass die Gasblase mit dem Blutstrom in den intrakorporalen Blutkreislauf des Patienten oder des Tieres zurückgeführt wird und dort zu einem lebensbedrohlichen Zustand oder sogar zum Tod führt.

Das hier beschriebene, erfindungsgemäße System 100 ist dabei für einen Blutstrom in einem Bereich von 0,05 bis 5I pro Minute, insbesondere für einen Blutstrom in einem Bereich von 0,1 bis 3I, insbesondere für einen Bereich von 0,2 bis 1I pro Minute, insbesondere für einen Bereich von 0,2 bis 0,5I pro Minute ausgelegt.

Zur Vermeidung von Komplikationen können die mit dem Blutstrom in Kontakt kommenden Oberflächen der Lumina des Systems 100 mit biokompatiblen und zumindest teilweise mit wenigstens einer funktionalen Beschichtung versehen sein, insbesondere mit einer antibakteriellen, gerinnungshemmenden und/oder anti-inflammatorischen Beschichtung.

Bei dem in Fig. 1 dargestellten ersten Ausführungsbeispiel eines erfindungsgemäßen Systems 100 zur extrakorporalen Blutbehandlung ist die Adsorbervorrichtung A in Blutflussrichtung vor der Dialysevorrichtung D angeordnet, wobei die Dialysevorrichtung D wiederum vor der Gasaustauschvorrichtung G in Blutflussrichtung angeordnet ist. Diese Anordnung hat den Vorteil, dass der Adsorbervorrichtung A ein unverdünnter Blutstrom zugeführt werden kann, wodurch eine hohe Effektivität der Adsorptionsbehandlung sichergestellt werden kann. Ferner kann es in der Adsorbervorrichtung A zu einer unerwünschten, unspezifischen Bindung von Ionen oder pH-Verschiebungen kommen, welche durch eine blutstromabwärts durchgeführte Dialysebehandlung mit einer in Fig. 1 dargestellten Reihenfolge der einzelnen Blutbehandlungsvorrichtung A, D und G durch die Dialysevorrichtung D ausgeglichen werden können.

Ferner kann die Dialysevorrichtung D bei dieser Anordnung in Blutflussrichtung nach der Adsorbervorrichtung A als weiteres Sicherheitssystem gegen einen unerwünschten Partikeleintrag aus der blutstromaufwärts angeordneten Adsorbervorrichtung A wirken.

Die Anordnung des Gasaustauschvorrichtung G nach der Adsorbervorrichtung A in Blutflussrichtung hat den Vorteil, dass eine Anreicherung des Blutstroms mit Kohlendioxid (CO2) durch die Zufuhr des Substituats nach der Adsorbervorrichtung A (welche bei dem in Fig. 1 gezeigten Ausführungsbeispiel über den vierten Einlass 4 nach der Adsorbervorrichtung und vor der Dialysevorrichtung erfolgen kann) sowie durch eine möglicherweise mit Kohlendioxid (CO2) beladene Dialysierflüssigkeit (welche dem System 100 in diesem Ausführungsbeispiel über den fünften Einlass 6 zugeführt werden kann) mithilfe der Gasaustauschvorrichtung G vor der Rückführung des behandelten Blutstroms in den intrakorporalen Blutkreislauf des Patienten bzw. des Tieres ausgeglichen werden kann.

Ist bei einem erfindungsgemäßen System zur extrakorporalen Blutbehandlung, bei welchem die Blutbehandlungsvorrichtungen wie anhand von Fig. 1 beschrieben angeordnet sind, die Dialysevorrichtung D statt zur Hämodialyse zur Hämodiafiltration ausgebildet, kann dem Blutstrom über den Einlass 4 bevorzugt auch ein Substituat, insbesondere eine zusätzliche Menge an Substituat, zum Ausgleich des Flüssigkeitsverlustes in der Dialysevorrichtung D zugeführt werden. Alternativ oder zusätzlich kann das System auch einen weiteren Einlass in Blutflussrichtung nach der Dialysevorrichtung D zur Zugabe des Substituats zum Ausgleich des Flüssigkeits- und/oder Volumenverlustes in der Adsorbervorrichtung A und/oder der Dialysevorrichtung D aufweisen.

Um eine flexible Anpassung des erfindungsgemäßen Systems 100 an die jeweilige, erforderliche Blutbehandlung zu ermöglichen, weisen die einzelnen Blutbehandlungsvorrichtungen A, D und G des erfindungsgemäßen Systems 100 jeweils austauschbare Behandlungsmodule auf, welche jeweils die komplette Behandlungsstrecke umfassen und einfach wie ein Ersatzteil ausgewechselt werden können. Auf diese Weise lässt sich das erfindungsgemäße System 100 einfach und schnell an die jeweilige, erforderliche Behandlung anpassen. So kann beispielsweise die Adsorbervorrichtung A schnell und einfach beispielsweise von einer Adsorbervorrichtung A zur Endotoxin-Adsorption in eine Adsorbervorrichtung A zur Zytokinadsorption umkonfiguriert werden, für welche je nach Anwendungsfall spezifisch ausgebildete Adsorber-Behandlungsmodule erforderlich sind.

Entsprechend kann durch einen Wechsel des jeweiligen Dialyse-Behandlungsmoduls die Dialysevorrichtung D des erfindungsgemäßen Systems 100 von einer zur Hämodialyse ausgebildeten Dialysevorrichtung D in eine zur Hämofiltration oder Hämodiafiltration ausgebildeten Dialysevorrichtung D umkonfiguriert werden.

Auf die gleiche Weise kann auch die Gasaustauschvorrichtung G des erfindungsgemäßen Systems 100 angepasst werden, wobei je nach erforderlicher Behandlung ein Gasaustausch-Behandlungsmodul eingesetzt werden kann, welches lediglich zur reinen CO2-Entfernung aus dem Blutstrom ausgebildet ist oder ein Gasaustausch-Behandlungsmodul, welches zusätzlich die Möglichkeit der Anreicherung des zu behandelnden Blutstroms mit Sauerstoff (02) oder einem anderen Gas oder einem Gasgemisch ermöglicht.

Vorzugsweise können die einzelnen Ein- und Auslässe, insbesondere hinsichtlich ihrer Anordnung innerhalb des Systems, insbesondere hinsichtlich ihrer Anordnung vor und/oder nach den jeweiligen Blutbehandlungsvorrichtungen, ebenfalls angepasst und/oder umkonfiguriert werden.

Dadurch kann das System 100 für jede Behandlung speziell konfiguriert werden. Ferner können beim Auftreten von Clotting oder dergleichen die einzelnen Behandlungsmodule schnell und einfach ausgetauscht werden. Darüber hinaus kann auf diese Weise auf besonders einfache Art und Weise die Bereitstellung eines sterilen Systems 100 zur Blutbehandlung sichergestellt werden, da vor Beginn der Behandlung eines neuen Patienten bzw. eines neuen Tieres sämtliche, mit dem Blutstrom in Kontakt kommenden Komponenten, insbesondere die jeweiligen Blutbehandlungsmodule und deren Schlauchverbindungen auf einfache Art und Weise ausgetauscht werden können.

Fig. 2 zeigt ein zweites Ausführungsbeispiel eines erfindungsgemäßen Systems 200 zur extrakorporalen Blutbehandlung, wobei Komponenten mit gleicher Funktionalität gleiche Bezugszeichen aufweisen.

Das in Fig. 2 ebenfalls lediglich schematisch dargestellte zweite Ausführungsbeispiel eines zweiten erfindungsgemäßen Systems 200 zur extrakorporalen Blutbehandlung ist vom Aufbau her grundsätzlich ähnlich zu dem anhand von Fig. 1 beschriebenen ersten Ausführungsbeispiel eines erfindungsgemäßen Systems 100 zur extrakorporalen Blutbehandlung, unterscheidet sich jedoch dahingehend von dem anhand von Fig. 1 beschriebenen erfindungsgemäßen System 100, dass die Reihenfolge der drei Blutbehandlungsvorrichtungen A, D und G bei dem in Fig. 2 dargestellten erfindungsgemäßen System 200 eine andere ist und das ferner die Dialysevorrichtung D nicht zur Hämodialyse ausgebildet ist und dementsprechend keinen Dialysator aufweist, sondern nur zur Hämofiltration und dementsprechend einen Hämofilter aufweist.

Aus diesem Grund weist das System 200 keinen fünften Einlass 6 zur Zufuhr eines Dialysats auf, da die Hämofiltration ohne die Zufuhr einer zusätzlichen Dialysierflüssigkeit auskommt und lediglich ein bei der Hämofiltration entstehendes Effluent abzuführen ist, welches bei dem in Fig. 2 dargestellten System ebenfalls über den zweiten Auslass 5 abgeführt werden kann.

Die in Fig. 2 dargestellte Ausgestaltung eines erfindungsgemäßen Systems 200 zur extrakorporalen Blutbehandlung, bei welchem die Dialysevorrichtung D in Blutflussrichtung vor der Adsorbervorrichtung A angeordnet ist und somit die Dialysevorrichtung D vor der Adsorbervorrichtung A mit dem zu behandelnden Blutstrom durchströmt wird, hat den Vorteil, dass durch einen Entzug der Filtrationsmenge (Effluent) im Hämofilter der Dialysevorrichtung D der Blutstrom aufkonzentriert wird und somit der Adsorbervorrichtung A ein stärker konzentrierter Blutstrom zugeführt werden kann gegenüber dem erfindungsgemäßen System 100 aus Fig. 1. Dadurch kann eine höhere Effektivität der Adsorptionsbehandlung erreicht werden. Folglich kann mit dem in Fig. 2 dargestellten erfindungsgemäßen System 200 eine verbesserte Adsorptionsbehandlung erreicht werden.

Durch die in Blutflussrichtung nachgeschaltete Anordnung der Gasaustauschvorrichtung G können auch bei dem in Fig. 2 dargestellten zweiten Ausführungsbeispiel eines erfindungsgemäßen Systems eine unerwünschte Aufladung bzw. Anreicherung des Blutmassenstroms mit Kohlendioxid (CO2) infolge der Zugabe eines CO2-beladenen Substituats über den vierten Einlass 4 vor der Rückführung in den intrakorporalen Blutkreislauf des Patienten oder des zu behandelnden Tieres ausgeglichen werden.

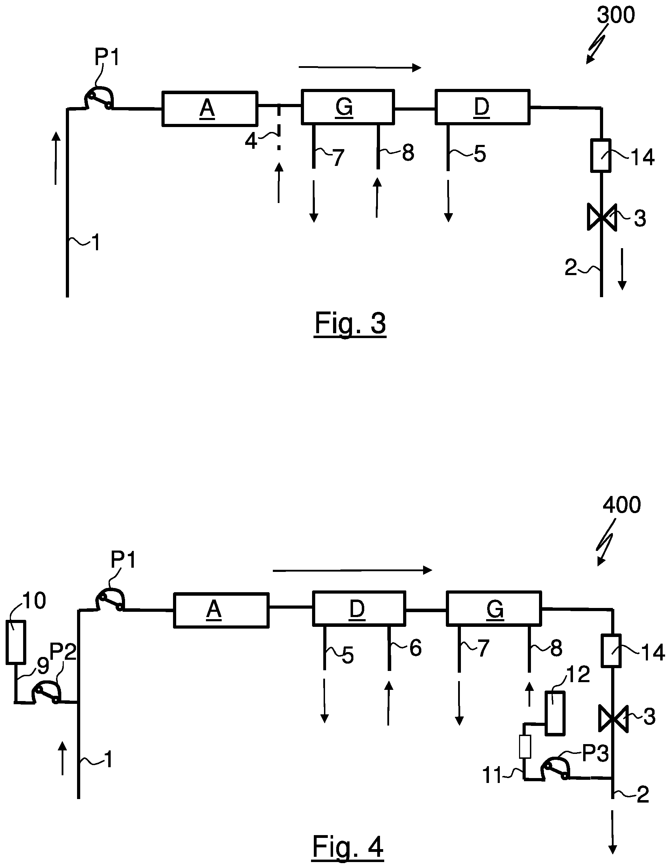

Durch die Zufuhr eines stärker konzentrierten Blutstroms in die Adsorbervorrichtung A steigt möglicherweise das Clotting-Risiko, insbesondere in der Adsorbervorrichtung A. Durch die jeweils unmittelbar vor und unmittelbar nach den drei Blutbehandlungsvorrichtungen A, D und G jeweils vorgesehenen Drucksensoreinrichtungen kann auftretendes Clotting jedoch schnell und zuverlässig erkannt werden und insbesondere durch die zusätzliche Zugabe eines Antikoagulants in den Blutstrom, beispielsweise durch die Zugabe von Citrat über den Einlass 9 (siehe Fig. 4), weitgehend verhindert werden. Ferner kann, sollte Clotting auftreten, die jeweilige Adsorbervorrichtung A bzw. können die jeweiligen, von Clotting betroffenen Blutbehandlungsvorrichtungen A, D und/oder G bzw. deren jeweiliges, eine Behandlungsstrecke bildendes Behandlungsmodul ausgetauscht werden.

Fig. 3 zeigt ein drittes Ausführungsbeispiel eines erfindungsgemäßen Systems 300 zur extrakorporalen Blutbehandlung, wobei dieses System 300 grundsätzlich ähnlich aufgebaut ist wie die beiden zuvor beschriebenen erfindungsgemäßen Systeme 100 und 200, sich jedoch ebenfalls in der Reihenfolge der Anordnung der einzelnen Blutbehandlungsvorrichtungen A, G und D von den beiden zuvor beschriebenen Ausführungsbeispielen erfindungsgemäßer Systeme 100 und 200 zur extrakorporalen Blutbehandlung unterscheidet.

Bei dem in Fig. 3 gezeigten dritten Ausführungsbeispiel eines erfindungsgemäßen Systems 300 zur Blutbehandlung wird, wie bei dem in Fig. 1 dargestellten erfindungsgemä-ßen System 100, die Adsorbervorrichtung A ebenfalls als erstes von dem zu behandelnden Blutmassenstrom durchströmt. Anschließend jedoch wird zunächst die Gasaustauschvorrichtung G durchströmt und erst nachgelagert die Dialysevorrichtung D.

Da die Dialysevorrichtung D bei dem in Fig. 3 dargestellten Ausführungsbeispiel eines erfindungsgemäßen Systems 300 ebenfalls lediglich zur Hämofiltration ausgebildet ist und nicht zur Hämodialyse, kann es nach der Gasaustauschvorrichtung G nicht mehr zur Aufladung des Blutstroms aufgrund eines Austauschs mit einem CO2 beladenen Dialysats kommen, da der diese Dialysevorrichtung kein Dialysat zugeführt wird, da dieses zur Hämofiltration nicht erforderlich ist.

Durch die Anordnung der Gasaustauschvorrichtung G nach der Adsorbervorrichtung A ist sichergestellt, dass eine möglicherweise unerwünschte CO2-Aufladung des Blutmassenstroms durch die Zufuhr eines CO2 beladenen Substituts über den vierten Einlass 4 ausgeglichen werden kann. Durch die der Adsorbervorrichtung A nachgeschaltete Anordnung der Dialysevorrichtung D in Blutflussrichtung kann auch in diesem Fall die Dialysevorrichtung D als sicherheitsrelevante Filterstufe gegen einen unerwünschten Partikeleintrag in der blutstromaufwärts angeordneten Adsorbervorrichtung A wirken. Ferner können möglicherweise in der Adsorbervorrichtung A entstandene, unerwünschte, unspezifische, zusätzliche lonenbindungen und/oder pH-Wert-Verschiebungen mittels der Dialysevorrichtung D auch mit dieser Anordnung bzw. mit diesem erfindungsgemäßen System 300 ausgeglichen werden. Darüber hinaus wird durch den der Gasaustauschvorrichtung G nachgelagerten Hämofilter im Gasaustauscher G ein Staudruck erzeugt, der vorteilhaft auf die Funktion des Gasaustauschers G wirkt.

Fig. 4 zeigt ein viertes Ausführungsbeispiel eines erfindungsgemäßen Systems 400 zur extrakorporalen Blutbehandlung, wobei dieses Ausführungsbeispiel eine besonders bevorzugte Ausgestaltung eines erfindungsgemäßen Systems zur extrakorporalen Blutbehandlung darstellt und ebenfalls auf dem anhand von Fig. 1 beschriebenen, ersten Ausführungsbeispiel eines erfindungsgemäßen Systems 100 basiert. Zusätzlich zu dem anhand von Fig. 1 beschriebenen erfindungsgemäßen System 100 weist dieses System 400 einen zweiten Einlass 9 sowie einen dritten Einlass 11 auf, über welchen jeweils eine Zusammensetzung dem Blutstrom zugeführt werden kann.

Dabei kann über den zweiten Einlass 9 mithilfe einer zweiten Pumpe P2 dem zu behandelnden Blutmassenstrom eine erste Zusammensetzung, welche vorzugsweise von einem Beutel 10 aufgenommen ist, zugeführt werden, wobei das System 400 in diesem Fall derart ausgestaltet ist, dass die erste Zusammensetzung dem Blutstrom unmittelbar nach der Entnahme aus dem intrakorporalen Blutkreislauf des zu behandelnden Patienten oder des zu behandelnden Tieres zugeführt werden kann, insbesondere in Blutflussrichtung noch vor der ersten Blutpumpe P1 und insbesondere vor der ersten Blutbehandlungsvorrichtung, die von dem zu behandelnden Blustrom durchströmt wird.

Insbesondere ist das System 400 dabei dazu ausgebildet über den zweiten Einlass 9 dem zu behandelnden Blutstrom mithilfe der zweiten Pumpe P2 eine flüssige Citratlösung als Antikoagulant zuzuführen.

Über den dritten Einlass 11 kann dem System eine zweite Zusammensetzung, insbesondere eine Kalziumlösung zum Ausgleich des bei der Hämodialyse in der Dialysevorrichtung D entstandenen Kalziumverlustes, mithilfe einer dritten Pumpe P3 zugeführt werden. Bei diesem, in Fig. 4 dargestellten vierten Ausführungsbeispiel eines erfindungsgemäßen Systems 400 zur extrakorporalen Blutbehandlung erfolgt die Zufuhr der zweiten Zusammensetzung über den dritten Einlass 11 dabei unmittelbar vor der Rückführung des behandelten Blutstroms in den Patienten, insbesondere nach dem Absperrventil 3. Selbstverständlich ist es auch möglich den dritten Einlass in Blutflussrichtung vor dem Absperrventil 3 anzuordnen.

Das in Fig. 4 dargestellte erfindungsgemäße System 400 eignet sich dabei insbesondere zur Behandlung in einem extrakorporalen Blutkreislauf mit einem veno-venösen Zugang, d.h. insbesondere zur CVVHD, bei welcher der zu behandelnde Blutstrom aus einer Vene des zu behandelnden Patienten oder Tieres entnommen wird und der behandelte Blutstrom in eine Vene zurückgeführt wird.

Ist bei einem erfindungsgemäßen System zur extrakorporalen Blutbehandlung, bei welchem die Blutbehandlungsvorrichtungen wie anhand von Fig. 4 beschrieben angeordnet sind, die Dialysevorrichtung D statt zur Hämodialyse zur Hämodiafiltration ausgebildet, kann dem Blutstrom vorzugsweise über einen zusätzlichen Einlass 4 in Blutflussrichtung nach der Adsorbervorrichtung A und vor der Dialysevorrichtung D bevorzugt ein Substituat zum Ausgleich des Flüssigkeitsverlustes in der Adsorbervorrichtung A und/oder der Dialysevorrichtung D zugeführt werden. Alternativ oder zusätzlich kann das System auch einen Einlass 4 in Blutflussrichtung nach der Dialysevorrichtung D zur Zugabe des Substituats zum Ausgleich des Flüssigkeits- und/oder Volumenverlustes in der Adsorbervorrichtung A und/oder der Dialysevorrichtung D aufweisen. Ein solches System eignet sich insbesondere zur post-CVVHDF.

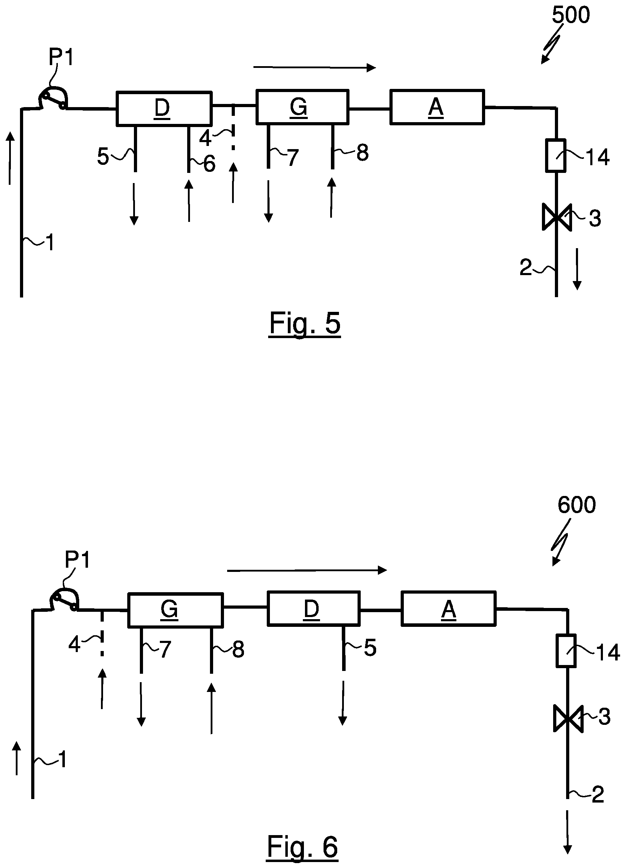

Fig. 5 zeigt ein fünftes Ausführungsbeispiel eines erfindungsgemäßen Systems 500 zur extrakorporalen Blutbehandlung, welches ebenfalls auf dem in Fig. 1 dargestellten erfindungsgemäßen System 100 basiert, sich jedoch dahingehend von dem System 100 aus Fig. 1 unterscheidet, dass die Adsorbervorrichtung A nach der Gasaustauschvorrichtung G angeordnet ist, insbesondere nach der Gasaustauschvorrichtung G und nach der Dialysevorrichtung D. Diese Anordnung hat den Vorteil, dass durch den erhöhten, an der Adsorbervorrichtung A entstehenden Staudruck das Druckgefälle in der Gasaustauschvorrichtung G an der Gasaustauschmembran steigt, wodurch ein verbesserter Gasaustausch erreicht werden kann.

Das Substituat zum Ausgleich des Volumenverlustes in der Adsorbervorrichtung A wird dabei vorzugsweise, wie in Fig. 5 abgebildet, über den Einlass 4 vor der Gasaustauschvorrichtung G dem Blutstrom zugegeben. Alternativ oder zusätzlich ist auch eine Zugabe vor der Dialysevorrichtung D in vorteilhafter Weise möglich. Dadurch kann eine unerwünschte CO2-Aufladung durch das Substituat mittels der nachfolgend angeordneten Gasaustauschvorrichtung G ausgeglichen werden.

Fig. 6 zeigt ein sechstes Ausführungsbeispiel eines erfindungsgemäßen Systems 600 zur extrakorporalen Blutbehandlung, welches auf dem in Fig. 5 dargestellten erfindungsgemäßen System 500 basiert, sich jedoch dahingehend von dem System 500 aus Fig. 5 unterscheidet, dass die Gasaustauschvorrichtung G in diesem Fall vor der Dialysevorrichtung D angeordnet ist, welche in diesem Fall nur zur Hämofiltration und nicht zur Hämodialyse ausgebildet ist.

Diese Anordnung hat den Vorteil, dass der Staudruck vor der Dialysevorrichtung D und vor der Adsorbervorrichtung A jeweils erhöhend auf das Druckgefälle innerhalb der Gasaustauschvorrichtung G an der Gasaustauschmembran wirkt, wodurch die Effizienz des Gasaustausches gesteigert werden kann.

Vorzugsweise wird das Substituat zum Ausgleich des Volumenverlustes in der Adsorbervorrichtung A ebenfalls über den Einlass 4 vor der Gasaustauschvorrichtung G dem Blutstrom zugegeben, so dass eine unerwünschte CO2-Aufladung durch das Substituat mittels der nachfolgend angeordneten Gasaustauschvorrichtung G ausgeglichen werden kann.

Dadurch, dass die Dialysevorrichtung D zur Hämofiltration ausgebildet ist, welche ohne ein Dialysat auskommt, kann eine unerwünschte CO2-Aufladung durch das Dialysat vermieden werden.

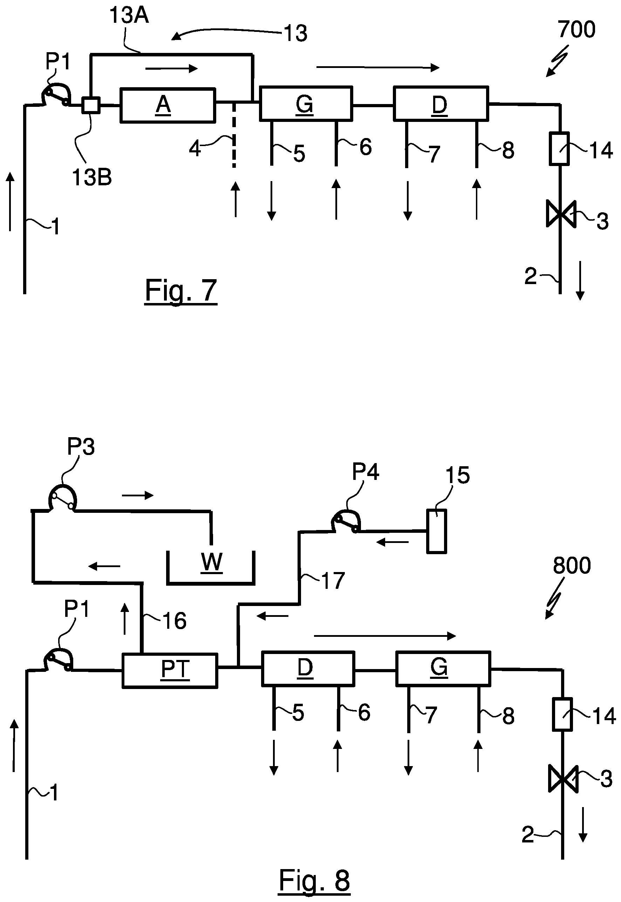

Fig. 7 zeigt ein siebtes Ausführungsbeispiel eines erfindungsgemäßen Systems 700 zur extrakorporalen Blutbehandlung, welches ebenfalls auf dem in Fig. 1 dargestellten erfindungsgemäßen System 100 basiert, bei welchem jedoch zusätzlich eine Bypasseinrichtung 13 vorhanden ist mit einer Bypassleitung 13A sowie einem Bypassventil 13B, welches eine Umleitung des zu behandelnden Blutstroms um die Adsorbervorrichtung A ermöglicht. Dieses System 700 hat den Vorteil, dass wahlweise eine Blutbehandlung mit oder ohne Adsorptionsbehandlung möglich ist. Dadurch wird die Flexibilität hinsichtlich der Einsatzmöglichkeiten eines erfindungsgemäßen Systems erheblich gesteigert, da nicht in allen Fällen zur Blutbehandlung jeweils eine Adsorptionsbehandlung angezeigt ist oder ein geclotteter oder ein nahezu vollständig beladener, bzw. gesättigter Adsorber umgangen werden kann.

Das Bypassventil 13B ist dabei vorzugsweise derart ausgebildet, dass ein zu behandelnder Blutstrom jeweils vollständig entweder durch die dem Bypassventil 13B in Blutflussrichtung nachgeschaltete Adsorbervorrichtung geführt wird oder vollständig über die Bypassleitung 13A an der Adsorbervorrichtung A vorbei.

In einer besonders vorteilhaften Ausgestaltung eines erfindungsgemäßen Systems weist das System für jede der Blutbehandlungsvorrichtungen A, D, G eine entsprechend ausgebildete Bypasseinrichtung auf, so dass mit einem erfindungsgemäßen System wahlweise eine Adsorptionsbehandlung, und/oder ein Gasaustausch und/oder eine Dialysebehandlung möglich ist.

Fig. 8 zeigt ein achtes Ausführungsbeispiel eines erfindungsgemäßen Systems 800, welches auf dem System 100 aus Fig. 1 basiert, wobei bei diesem Ausführungsbeispiel die erste Blutbehandlungsvorrichtung anstelle einer Adsorbervorrichtung A eine Plasmatrennvorrichtung PT in Form eines Plasmafilters PT ist, über welche mithilfe einer weiteren, insbesondere dritten, Pumpe P3, abgetrenntes Blutplasma über eine Leitung 16, die einen vierten Auslass zur Abfuhr von abgetrenntem Blutplasma bildet, einem Blutplasma-Entsorgungsbehälter W zugeführt werden kann.

Über eine Leitung 17, welche einen siebten Einlass 17 bildet, kann dem Blutstrom als Ersatz für die abgeführte Menge an Blutplasma frisches Plasma zugeführt werden, insbesondere mithilfe einer weiteren, insbesondere vierten Pumpe P4.

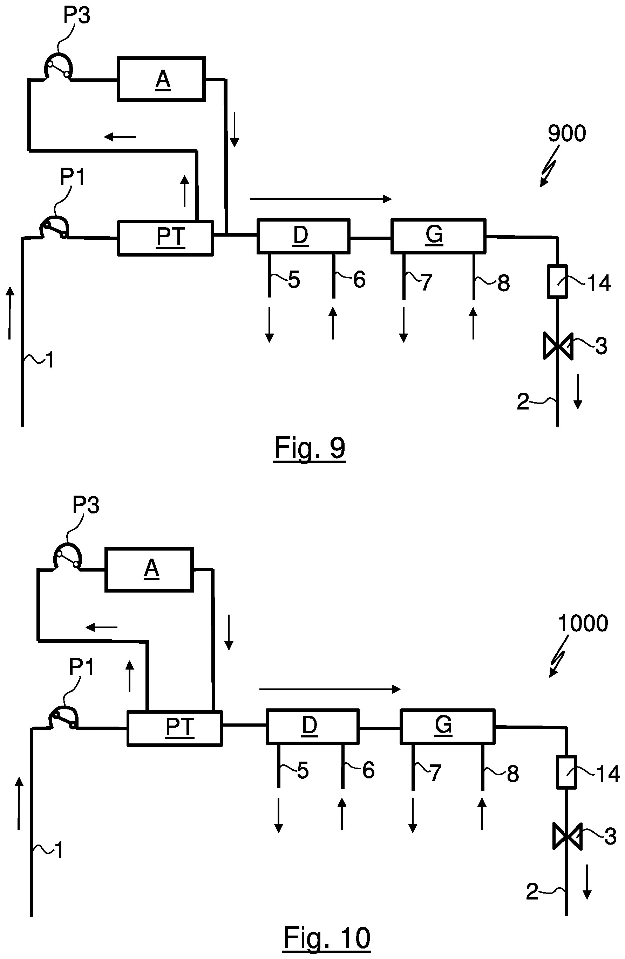

Fig. 9 zeigt ein neuntes Ausführungsbeispiel eines erfindungsgemäßen Systems 900, welches auf dem System 800 aus Fig. 8 basiert, wobei bei diesem Ausführungsbeispiel, das abgetrennte Plasma nicht einem Blutplasma-Entsorgungsbehälter W zugeführt wird, sondern mittels einer Schlauchleitung, insbesondere mittels einer Bypassleitung, durcheine Blutbehandlungsvorrichtung in Form einer Adsorbervorrichtung A hindurch geführt wird, wobei das behandelte Plasma nach der Adsorptionsbehandlung dem übrigen Blutstrom wieder zugeführt wird, insbesondere in die Hauptleitung.

Fig. 10 zeigt ein zehntes Ausführungsbeispiel eines erfindungsgemäßen Systems 1000, welches auf dem System 900 aus Fig. 9 basiert, wobei bei diesem Ausführungsbeispiel, das abgetrennte Plasma nach der Adsorptionsbehandlung nicht dem übrigen Blutstrom zugeführt wird, sondern der Plasmatrennvorrichtung PT zur Rezirkulation in einem Bypass-Kreislauf. Dadurch kann eine verbesserte Plasmatrennung und/oder Adsorptionsbehandlung erreicht werden.

### Bezugszeichenliste:

- 100, 200, 300,: erfindungsgemäßes System zur extrakorporalen Blutbehandlung
- 400, 500, 600, 700, 800, 900, 1000 1: Zuführleitung (erster Einlass)
- 2: Rückführleitung (erster Auslass)
- 3: Absperrventil
- 4: vierter Einlass zur Zufuhr eines Substituts in den Blutstrom
- 5: zweiter Auslass zur Abfuhr eines Effluents aus der Dialysevorrichtung
- 6: fünfter Einlass zur Zufuhr eines Dialysats in die Dialysevorrichtung
- 7: dritter Auslass zur Abfuhr von CO2 aus der Gasaustauschvorrichtung
- 8: sechster Einlass zur Zufuhr eines Gases oder Gasgemisches in die Gasaustauschvorrichtung
- 9: zweiter Einlass zur Zufuhr einer ersten Zusammensetzung in den Blutstrom
- 10: Beutel, gefüllt mit einer ersten Zusammensetzung
- 11: dritter Einlass zur Zufuhr einer zweiten Zusammensetzung in den Blutstrom
- 12: Beutel, gefüllt mit einer zweiten Zusammensetzung
- 13: Bypasseinrichtung
- 13A: Bypassleitung
- 13B: Bypassventil
- 14: Gasblasen-Detektionseinrichtung
- 15: Beutel, gefüllt mit frischem Blutplasma
- 16: vierter Auslass zur Abfuhr von abgetrenntem Blutplasma
- 17: siebter Einlass zur Zufuhr von frischem Blutplasma

- A: Adsorbervorrichtung
- D: Dialysevorrichtung
- G: Gasaustauschvorrichtung
- PT: Plasmatrennvorrichtung
- P1: erste Pumpe, Blutpumpe
- P2: zweite Pumpe
- P3: dritte Pumpe
- P4: vierte Pumpe
- P5: fünfte Pumpe
- W: Blutplasma-Entsorgungsbehälter

## Patentansprüche

1. System (100, 200, 300, 400, 500, 600, 700, 800, 900, 1000) zur extrakorporalen Blutbehandlung, wobei das System (100, 200, 300, 400, 500, 600, 700, 800, 900, 1000)
- einen ersten Einlass (1) zum Einbringen eines zu behandelnden Blutstroms in das System (100, 200, 300, 400, 500, 600, 700, 800, 900, 1000),
- wenigstens eine erste Blutbehandlungsvorrichtung (A, PT),
- eine zweite Blutbehandlungsvorrichtung (D),
- eine dritte Blutbehandlungsvorrichtung (G) und
- einen ersten Auslass (2) zum Ausbringen eines behandelten Blutstroms aus dem System (100, 200, 300, 400, 500, 600,700, 800, 900, 1000) aufweist,
wobei die erste Blutbehandlungsvorrichtung (A, PT) eine Adsorbervorrichtung (A) zur Entfernung wenigstens eines körperfremden und/oder wenigstens eines körpereigenen Pathogens und/oder eine Plasmatrennvorrichtung (PT) zum Trennen von Blutplasma von den übrigen Blutbestandteilen ist oder aufweist,
wobei die zweite Blutbehandlungsvorrichtung (D) als Dialysevorrichtung (D) ausgebildet ist, insbesondere als Dialysevorrichtung (D) zur Nierenersatztherapie, und
wobei die dritte Blutbehandlungsvorrichtung (G) als Gasaustauschvorrichtung (G) ausgebildet ist, insbesondere als Gasaustauschvorrichtung (G) zur zumindest teilweisen CO2-Entfernung aus einem die Gasaustauschvorrichtung (G) durchströmenden Blutstrom und/oder zur Zufuhr eines Gases oder Gasgemisches in einen die Gastaustauschvorrichtung (G) durchströmenden Blutstrom, und
wobei die drei Blutbehandlungsvorrichtungen (A und/oder PT, D, G) in einem funktionsgemäßen Anwendungszustand des Systems (100, 200, 300, 400, 500, 600, 700, 800, 900, 1000) bezogen auf eine Blutflussrichtung eines zu behandelnden Blutstroms zwischen dem ersten Einlass (1) und dem ersten Auslass (2) des Systems sequenziell in Reihe geschaltet sind und nacheinander von einem zu behandelnden Blutstrom extrakorporal durchströmbar sind.

2. System (100, 200, 300, 400, 500, 600, 700, 800, 900, 1000) nach Anspruch 1, **dadurch gekennzeichnet, dass** die erste Blutbehandlungsvorrichtung (A) eine zur Endotoxin-, Zytokin und/oder zur Immunadsorption ausgebildete Adsorbervorrichtung (A) ist oder aufweist, wobei die Adsorbervorrichtung (A) insbesondere zur Entfernung von wenigstens einem Medikament und/oder Arzneimittelwirkstoff und/oder wenigstens einem Pflanzengift und/oder wenigstens einem organischen Gift und/oder wenigstens einer anderen toxischen Substanz und/oder zur Entfernung von Bakterien, Viren, Pilzen und/oder anderen Organismen und/oder wenigstens eines Immunkomplexes und/oder wenigstens eines Immunglobulins und/oder wenigstens einer Substanz der infllammatorischen Antwort des Körpers und/oder Antikörpern und/oder wenigstens eines pathogen-assoziierten molekularen Musters und/oder wenigstens eines Alarmins ausgebildet ist.

3. System (100, 200, 300, 400, 500, 600, 700, 800, 900, 1000) nach Anspruch 1 oder 2, **dadurch gekennzeichnet, dass** das System (100, 200, 300, 400, 500, 600, 700, 800, 900, 1000) wenigstens eine erste Pumpe (P1), insbesondere eine als Blutpumpe ausgebildete Pumpe (P1), zur Förderung wenigstens eines Anteils eines zu behandelnden Blutstroms aufweist, wobei die erste Pumpe (P1) vorzugsweise in Blutflussrichtung zwischen dem ersten Einlass (1) und der ersten der drei Blutbehandlungseinrichtungen (A, PT, D, G) in Blutflussrichtung angeordnet ist und insbesondere zur Förderung des gesamten zu behandelnden Blutstroms ausgebildet ist.

4. System (400) nach einem der vorgenannten Ansprüche, **dadurch gekennzeichnet, dass** das System (400) einen weiteren, insbesondere zweiten, Einlass (9) zur Zugabe einer ersten Zusammensetzung in den Blutstrom aufweist, insbesondere in den zu behandelnden Blutstrom, wobei dieser weitere Einlass (9) vorzugsweise in Blutflussrichtung derart angeordnet ist, dass die Zusammensetzung dem Blutstrom in Blutflussrichtung vor der ersten Pumpe (P1) zuführbar ist und/oder vor der ersten der drei Blutbehandlungsvorrichtungen (A, PT, D, G).

5. System (400) nach einem der vorgenannten Ansprüche, **dadurch gekennzeichnet, dass** das System (400) einen weiteren, insbesondere dritten, Einlass (11) zur Zugabe einer zweiten Zusammensetzung in den Blutstrom aufweist, insbesondere in den behandelten Blutstrom, wobei dieser weitere Einlass (11) vorzugsweise in Blutflussrichtung derart angeordnet ist, dass die Zusammensetzung dem Blutstrom in Blutflussrichtung nach der Dialysevorrichtung (D) zuführbar ist, insbesondere nach der letzten Blutbehandlungsvorrichtungen (A, PT, D, G) in Blutflussrichtung.

6. System (100, 200, 300, 400, 700, 800, 900, 1000) nach einem der vorgenannten Ansprüche, **dadurch gekennzeichnet, dass** die Adsorbervorrichtung (A) und/oder die Plasmatrennvorrichtung (PT) in Blutflussrichtung vor der Gasaustauschvorrichtung (G) oder nach der Gasaustauschvorrichtung (G) angeordnet ist.

7. System (100, 300, 400, 700, 800, 900, 1000) nach einem der vorgenannten Ansprüche, **dadurch gekennzeichnet, dass** die Adsorbervorrichtung (A) und/oder die Plasmatrennvorrichtung (PT) in Blutflussrichtung vor der Dialysevorrichtung (D) angeordnet ist, wobei die Dialysevorrichtung (D) vorzugsweise zur Hämodialyse ausgebildet ist und insbesondere einen Dialysator aufweist oder
**dadurch gekennzeichnet, dass** die Adsorbervorrichtung (A) und/oder die Plasmatrennvorrichtung (PT) in Blutflussrichtung nach der Dialysevorrichtung (D) angeordnet ist, wobei die Dialysevorrichtung (D) vorzugsweise zur Hämofiltration ausgebildet ist und insbesondere einen Hämofilter aufweist.

8. System (100, 200, 400, 500, 800, 900, 1000) nach einem der vorgenannten Ansprüche, **dadurch gekennzeichnet, dass** die Dialysevorrichtung (D) in Blutflussrichtung vor der Gasaustauschvorrichtung (G) angeordnet ist oder,
dass die Dialysevorrichtung (D) in Blutflussrichtung nach der Gasaustauschvorrichtung (G) angeordnet ist, wobei die Dialysevorrichtung (D) vorzugsweise zur Hämofiltration ausgebildet ist und einen Hämofilter aufweist.

9. System (100, 200, 300, 400, 500, 600, 700, 800, 900, 1000) nach einem der vorgenannten Ansprüche, **dadurch gekennzeichnet, dass** das System (100, 200, 300, 400, 500, 600, 700, 800, 900, 1000) wenigstens eine Drucksensoreinrichtung zur Ermittlung eines Strömungsdruckes des Blutstroms an einer definierten Stelle im System (100, 200, 300, 400, 500, 600, 700, 800, 900, 1000) aufweist, wobei vorzugsweise in Blutflussrichtung unmittelbar vor und/oder unmittelbar nach wenigstens einer Behandlungsstrecke einer Blutbehandlungsvorrichtung (A, PT, D, G) wenigstens eine Drucksensoreinrichtung angeordnet ist.

10. System (100, 200, 300, 400, 500, 600, 700, 800, 900, 1000) nach einem der vorgenannten Ansprüche, **dadurch gekennzeichnet, dass** das System (100, 200, 300, 400, 500, 600, 700, 800, 900, 1000) wenigstens eine Gasblasen-Detektionseinrichtung (14) zum Erkennen einer Gasblase im Blutstrom aufweist.

11. System (100, 200, 300, 400, 500, 600, 700, 800, 900, 1000) nach einem der vorgenannten Ansprüche, **dadurch gekennzeichnet, dass** bei wenigstens einer Blutbehandlungsvorrichtung (A, PT, D, G) eine Behandlungsstrecke zumindest teilweise, vorzugsweise vollständig, durch ein austauschbares Behandlungsmodul gebildet ist, insbesondere durch ein kartuschenartiges Behandlungsmodul.

12. System (700) nach einem der vorgenannten Ansprüche, **dadurch gekennzeichnet, dass** das System (700) wenigstens eine schaltbare Bypasseinrichtung (13) zur Umgehung wenigstens einer Blutbehandlungsvorrichtung (A, D, G) aufweist.

13. System (100, 200, 300, 400, 500, 600, 700, 800, 900, 1000) nach einem der vorgenannten Ansprüche, **dadurch gekennzeichnet, dass** wenigstens eine Komponente des Systems (100, 200, 300, 400, 500, 600, 700, 800, 900, 1000) an einer mit dem zu behandelnden Blutstrom in Kontakt kommenden Oberfläche eine biokompatible und vorzugsweise funktionale Beschichtung aufweist, insbesondere eine antibakterielle, gerinnungshemmende und/oder anti-inflammatorische Beschichtung.

14. Behandlungsvorrichtung zur extrakorporalen Blutbehandlung, **dadurch gekennzeichnet, dass** die Behandlungsvorrichtung ein System (100, 200, 300, 400, 500, 600, 700, 800, 900, 1000) aufweist, das nach einem der Ansprüche 1 bis 13 ausgebildet ist, wobei die erste, die zweite und die dritte Blutbehandlungsvorrichtung (A, PT, D, G) des Systems (100, 200, 300, 400, 500, 600, 700, 800, 900, 1000) in einem gemeinsamen Gehäuse angeordnet sind und/oder von einer gemeinsamen Basis aufgenommen sind.

## Claims

1. A system (100, 200, 300, 400, 500, 600, 700, 800, 900, 1000) for extracorporeal blood treatment, wherein the system (100, 200, 300, 400, 500, 600, 700, 800, 900, 1000) comprises:
- a first inlet (1) for introducing a bloodstream to be treated into the system (100, 200, 300, 400, 500, 600, 700, 800, 900, 1000),
- at least one first blood treatment apparatus (A, PT),
- one second blood treatment apparatus (D),
- one third blood treatment apparatus (G), and
- a first outlet (2) for discharging a treated bloodstream from the system (100, 200, 300, 400, 500, 600,700, 800, 900, 1000),
wherein the first blood treatment apparatus (A, PT) is or comprises an adsorber apparatus (A) for removing at least one exogenous and/or at least one endogenous pathogen and/or a plasma separator apparatus (PT) for separating blood plasma from the other blood components,
wherein the second blood treatment apparatus (D) is designed as a dialysis apparatus (D), in particular as a dialysis apparatus (D) for renal replacement therapy, and
wherein the third blood treatment apparatus (G) is designed as a gas exchange apparatus (G), in particular as a gas exchange apparatus (G) for at least partially removing CO2 from a stream of blood flowing through the gas exchange apparatus (G) and/or for supplying a gas or gas mixture into a stream of blood flowing through the gas exchange apparatus (G), and
wherein the three blood treatment apparatus (A and/or PT, D, G) are sequentially connected in series in a functional state of system (100, 200, 300, 400, 500, 600, 700, 800, 900, 1000) application between the first inlet (1) and the first outlet (2) of the system relative to a direction of blood flow of a bloodstream to be treated and can be consecutively perfused extracorporeally by a bloodstream to be treated.

2. The system (100, 200, 300, 400, 500, 600, 700, 800, 900, 1000) according to claim 1, **characterized in that** the first blood treatment apparatus (A) is or comprises an adsorber apparatus (A) designed for endotoxin adsorption, cytokine adsorption and/or immunoadsorption, wherein the adsorber apparatus (A) is in particular designed to remove at least one pharmaceutical and/or medical drug and/or at least one phytotoxin and/or at least one organic toxin and/or at least one other toxic substance and/or to remove bacteria, viruses, fungi and/or other organisms and/or at least one immune complex and/or at least one immunoglobulin and/or at least one inflammatory response substance of the body and/or antibodies and/or at least one pathogen-associated molecular pattern and/or at least one alarmin.

3. The system (100, 200, 300, 400, 500, 600, 700, 800, 900, 1000) according to claim 1 or 2, **characterized in that** the system (100, 200, 300, 400, 500, 600, 700, 800, 900, 1000) comprises at least one first pump (P1), in particular a pump (P1) designed as a blood pump, for pumping at least a portion of a bloodstream to be treated, wherein the first pump (P1) is preferably arranged between the first inlet (1) in the blood flow direction and the first of the three blood treatment apparatus (A, PT, D, G) in the blood flow direction and is in particular designed to pump the entire bloodstream to be treated.

4. The system (400) according to one of the preceding claims, **characterized in that** the system (400) comprises a further, in particular second, inlet (9) for the addition of a first compound into the bloodstream, in particular into the bloodstream to be treated, wherein this further inlet (9) is preferably arranged in the direction of blood flow such that the compound can be fed into the bloodstream upstream of the first pump (P1) and/or upstream of the first of the three blood treatment apparatus (A, PT, D, G).

5. The system (400) according to one of the preceding claims, **characterized in that** the system (400) comprises a further, in particular third, inlet (11) for the addition of a second compound into the bloodstream, in particular into the bloodstream to be treated, wherein this further inlet (11) is preferably arranged in the direction of blood flow such that the compound can be fed into the bloodstream downstream of the dialysis apparatus (D), in particular downstream of the last blood treatment apparatus (A, PT, D, G).

6. The system (100, 200, 300, 400, 700, 800, 900, 1000) according to one of the preceding claims, **characterized in that** the adsorber apparatus (A) and/or the plasma separator apparatus (PT) is/are arranged upstream or downstream of the gas exchange apparatus (G) in the blood flow direction.

7. The system (100, 300, 400, 700, 800, 900, 1000) according to one of the preceding claims, **characterized in that** the adsorber apparatus (A) and/or the plasma separator apparatus (PT) is/are arranged upstream of the dialysis apparatus (D) in the blood flow direction, wherein the dialysis apparatus (D) is preferably designed for hemodialysis and in particular comprises a dialyzer; or
**characterized in that** the adsorber apparatus (A) and/or the plasma separator apparatus (PT) is/are arranged downstream of the dialysis apparatus (D) in the blood flow direction, wherein the dialysis apparatus (D) is preferably designed for hemofiltration and in particular comprises a hemofilter.

8. The system (100, 200, 400, 500, 800, 900, 1000) according to one of the preceding claims, **characterized in that** the dialysis apparatus (D) is arranged upstream of the gas exchange apparatus (G) in the blood flow direction; or
**characterized in that** the dialysis apparatus (D) is arranged downstream of the gas exchange apparatus (G) in the blood flow direction, wherein the dialysis apparatus (D) is preferably designed for hemofiltration and comprises a hemofilter.

9. The system (100, 200, 300, 400, 500, 600, 700, 800, 900, 1000) according to one of the preceding claims, **characterized in that** the system (100, 200, 300, 400, 500, 600, 700, 800, 900, 1000) comprises at least one pressure sensor device for determining a bloodstream flow pressure at a defined point in the system (100, 200, 300, 400, 500, 600, 700, 800, 900, 1000), wherein at least one pressure sensor device is preferably arranged directly ahead of and/or directly after at least one treatment segment of a blood treatment apparatus (A, PT, D, G) in the blood flow direction.

10. The system (100, 200, 300, 400, 500, 600, 700, 800, 900, 1000) according to one of the preceding claims, **characterized in that** the system (100, 200, 300, 400, 500, 600, 700, 800, 900, 1000) comprises at least one gas bubble detection device (14) for detecting a gas bubble in the bloodstream.

11. The system (100, 200, 300, 400, 500, 600, 700, 800, 900, 1000) according to one of the preceding claims, **characterized in that** a treatment segment for at least one blood treatment apparatus (A, PT, D, G) is at least partially, preferably entirely, formed by an exchangeable treatment module, in particular by a cartridge-like treatment module.

12. The system (700) according to one of the preceding claims, **characterized in that** the system (700) comprises at least one switchable bypass device (13) for bypassing at least one blood treatment apparatus (A, D, G).

13. The system (100, 200, 300, 400, 500, 600, 700, 800, 900, 1000) according to one of the preceding claims, **characterized in that** at least one component of the system (100, 200, 300, 400, 500, 600, 700, 800, 900, 1000) has a biocompatible and preferably functional coating on a surface coming into contact with the bloodstream to be treated, in particular an antibacterial, coagulation-inhibiting and/or anti-inflammatory coating.

14. A treatment apparatus for extracorporeal blood treatment, **characterized in that** the treatment apparatus comprises a system (100, 200, 300, 400, 500, 600, 700, 800, 900, 1000) designed in accordance with one of claims 1 to 13, wherein the first, the second and the third blood treatment apparatus (A, PT, D, G) of the system (100, 200, 300, 400, 500, 600, 700, 800, 900, 1000) are arranged in a common housing and/or accommodated by a common base.

## Revendications

1. Système (100, 200, 300, 400, 500, 600, 700, 800, 900, 1000) pour le traitement extracorporel de sang, dans lequel le système (100, 200, 300, 400, 500, 600, 700, 800, 900, 1000) comporte
- une première entrée (1) pour l'introduction d'un flux sanguin à traiter dans le système (100, 200, 300, 400, 500, 600, 700, 800, 900, 1000),
- au moins un premier dispositif de traitement de sang (A, PT),
- un deuxième dispositif de traitement de sang (D),
- un troisième dispositif de traitement de sang (G) et
- une première sortie (2) pour l'évacuation d'un flux sanguin traité hors du système (100, 200, 300, 400, 500, 600, 700, 800, 900, 1000),
dans lequel le premier dispositif de traitement de sang (A, PT) est ou comporte un dispositif adsorbeur (A) pour l'élimination d'au moins un pathogène étranger au corps et/ou d'au moins un pathogène propre au corps et/ou un dispositif de séparation de plasma (PT) pour la séparation d'un plasma sanguin par rapport aux composants sanguins restants,
dans lequel le deuxième dispositif de traitement de sang (D) est conçu en tant que dispositif de dialyse (D), en particulier en tant que dispositif de dialyse (D) pour la thérapie de remplacement rénal, et
dans lequel le troisième dispositif de traitement de sang (G) est conçu en tant que dispositif d'échange gazeux (G), en particulier en tant que dispositif d'échange gazeux (G) pour l'élimination au moins partielle de CO2 depuis un flux sanguin s'écoulant à travers le dispositif d'échange gazeux (G) et/ou pour l'introduction d'un gaz ou d'un mélange gazeux jusque dans un flux sanguin s'écoulant à travers le dispositif d'échange gazeux (G), et
dans lequel les trois dispositifs de traitement de sang (A et/ou PT, D, G) sont, dans un état d'utilisation fonctionnellement conforme du système (100, 200, 300, 400, 500, 600, 700, 800, 900, 1000) et par rapport à une direction de flux sanguin d'un flux sanguin à traiter, montés séquentiellement en série entre la première entrée (1) et la première sortie (2) du système et peuvent successivement être traversés par écoulement et de façon extracorporelle par un flux sanguin à traiter.

2. Système (100, 200, 300, 400, 500, 600, 700, 800, 900, 1000) selon la revendication 1, **caractérisé en ce que** le premier dispositif de traitement de sang (A) est ou comporte un dispositif adsorbeur (A) conçu pour l'adsorption d'une endotoxine, d'une cytokine et/ou pour l'immuno-adsorption, dans lequel le dispositif adsorbeur (A) est en particulier conçu pour l'élimination d'au moins un médicament et/ou principe actif de remède et/ou d'au moins un poison végétal et/ou d'au moins un poison organique et/ou d'au moins une autre substance toxique et/ou pour l'élimination de bactéries, virus, champignons et/ou autres organismes et/ou d'au moins un complexe immun et/ou d'au moins une globuline immune et/ou d'au moins une substance de la réponse inflammatoire du corps et/ou des anticorps et/ou d'au moins un modèle moléculaire associé à un pathogène et/ou d'au moins une alarmine.

3. Système (100, 200, 300, 400, 500, 600, 700, 800, 900, 1000) selon la revendication 1 ou 2, **caractérisé en ce que** le système (100, 200, 300, 400, 500, 600, 700, 800, 900, 1000) comporte au moins une première pompe (P1), en particulier une pompe (P1) conçue en tant que pompe à sang, pour la stimulation d'au moins une partie d'un flux sanguin à traiter, dans lequel la première pompe (P1) est agencée de préférence dans la direction de flux sanguin entre la première entrée (1) et le premier des trois dispositifs de traitement de sang (A, PT, D, G) dans la direction de flux sanguin et est en particulier conçue pour la stimulation de la totalité du flux sanguin à traiter.

4. Système (400) selon l'une des revendications précédentes, **caractérisé en ce que** le système (400) comporte une autre, en particulier une deuxième, entrée (9), pour l'ajout d'une première composition dans le flux sanguin, en particulier dans le flux sanguin à traiter, dans lequel cette autre entrée (9) est agencée de préférence dans la direction de flux sanguin de sorte que la composition puisse être amenée au flux sanguin dans la direction de flux sanguin avant la première pompe (P1) et/ou avant le premier des trois dispositifs de traitement de sang (A, PT, D, G).

5. Système (400) selon l'une des revendications précédentes, **caractérisé en ce que** le système (400) comporte une autre, en particulier une troisième, entrée (11) pour l'ajout d'une seconde composition dans le flux sanguin, en particulier dans le flux sanguin traité, dans lequel cette autre entrée (11) est agencée de préférence dans la direction de flux sanguin de sorte que la composition puisse être amenée au flux sanguin dans la direction de flux sanguin après le dispositif de dialyse (D), en particulier après le dernier dispositif de traitement de sang (A, PT, D, G) dans la direction de flux sanguin.

6. Système (100, 200, 300, 400, 700, 800, 900, 1000) selon l'une des revendications précédentes, **caractérisé en ce que** le dispositif adsorbeur (A) et/ou le dispositif de séparation de plasma (PT) est agencé dans la direction de flux sanguin avant le dispositif d'échange gazeux (G) ou après le dispositif d'échange gazeux (G) .

7. Système (100, 300, 400, 700, 800, 900, 1000) selon l'une des revendications précédentes, **caractérisé en ce que** le dispositif adsorbeur (A) et/ou le dispositif de séparation de plasma (PT) est agencé dans la direction de flux sanguin avant le dispositif de dialyse (D), dans lequel le dispositif de dialyse (D) est conçu de préférence pour l'hémodialyse et comporte en particulier un dialyseur ou
est **caractérisé en ce que** le dispositif adsorbeur (A) et/ou le dispositif de séparation de plasma (PT) est agencé dans la direction de flux sanguin après le dispositif de dialyse (D), dans lequel le dispositif de dialyse (D) est conçu de préférence pour l'hémofiltration et comporte en particulier un hémofiltre.

8. Système (100, 200, 400, 500, 800, 900, 1000) selon l'une des revendications précédentes, **caractérisé en ce que** le dispositif de dialyse (D) est agencé dans la direction de flux sanguin avant le dispositif d'échange gazeux (G) ou,
**en ce que** le dispositif de dialyse (D) est agencé dans la direction de flux sanguin après le dispositif d'échange gazeux (G), dans lequel le dispositif de dialyse (D) est conçu de préférence pour l'hémofiltration et comporte un hémofiltre.

9. Système (100, 200, 300, 400, 500, 600, 700, 800, 900, 1000) selon l'une des revendications précédentes, **caractérisé en ce que** le système (100, 200, 300, 400, 500, 600, 700, 800, 900, 1000) comporte au moins un équipement de capteur de pression pour l'établissement d'une pression d'écoulement du flux sanguin en un emplacement défini dans le système (100, 200, 300, 400, 500, 600, 700, 800, 900, 1000), dans lequel au moins un équipement de détection de pression est agencé de préférence dans la direction de flux sanguin immédiatement avant et/ou immédiatement après au moins un tronçon de traitement d'un dispositif de traitement de sang (A, PT, D, G).

10. Système (100, 200, 300, 400, 500, 600, 700, 800, 900, 1000) selon l'une des revendications précédentes, **caractérisé en ce que** le système (100, 200, 300, 400, 500, 600, 700, 800, 900, 1000) comporte au moins un équipement de détection de bulle gazeuse (14) pour la détection d'une bulle gazeuse dans le flux sanguin.

11. Système (100, 200, 300, 400, 500, 600, 700, 800, 900, 1000) selon l'une des revendications précédentes, **caractérisé en ce que**, dans le cas d'au moins un dispositif de traitement de sang (A, PT, D, G), un tronçon de traitement est formé au moins partiellement, de préférence entièrement, par un module de traitement interchangeable, en particulier par un module de traitement en forme de cartouche.

12. Système (700) selon l'une des revendications précédentes, **caractérisé en ce que** le système (700) comporte au moins un équipement de contournement (13) qui peut être commuté pour le contournement d'au moins un dispositif de traitement de sang (A, D, G).

13. Système (100, 200, 300, 400, 500, 600, 700, 800, 900, 1000) selon l'une des revendications précédentes, **caractérisé en ce qu'**au moins une composante du système (100, 200, 300, 400, 500, 600, 700, 800, 900, 1000) comporte, sur une surface entrant en contact avec le flux sanguin à traiter, un revêtement biocompatible et de préférence fonctionnel, en particulier un revêtement antibactérien, anticoagulant et/ou anti-inflammatoire.

14. Dispositif de traitement pour le traitement extracorporel de sang, **caractérisé en ce que** le dispositif de traitement comporte un système (100, 200, 300, 400, 500, 600, 700, 800, 900, 1000) qui est conçu selon l'une des revendications 1 à 13, dans lequel le premier, le deuxième et le troisième dispositif de traitement de sang (A, PT, D, G) du système (100, 200, 300, 400, 500, 600, 700, 800, 900, 1000) sont agencés dans un logement commun et/ou sont reçus par une base commune.
